# EUROPEAN PATENT APPLICATION

(11) **EP 1 498 486 A1**
(43) Date of publication of application: **19.01.2005**
(21) Application number: 03745446.9
(22) Date of filing: 28.03.2003
(51) Int. Cl.: C12N 15/09, C12M 1/00, C07K 17/00, G01N 33/50

(54) **NUCLEIC ACID LIBRARY AND PROTEIN LIBRARY**

(30) Priority: 29.03.2002 JP 2002095285
(71) Applicant: PRECISION SYSTEM SCIENCE CO., LTD., Matsudo-shi, Chiba, 271-0064 (JP); National Institute of Advanced Industrial Science and Technology, Tokyo 100-8921 (JP)
(72) Inventor: TAJIMA, Hideji, Precision System Science Co. Ltd., Matsudo-shi, Chiba 271-0064 (JP); MACHIDA, Masayuki, AIST Tsuku Central 6, Tsukuba (JP); HAGIWARA, Youko, AIST Tsuku Central 6, Tsukuba (JP); KUNIHIRO, Sumiko, AIST Tsukuba Central 6, Tsukuba (JP)
(74) Representative: Bohnenberger, Johannes, Dr.
(86) International application number: PCT/JP2003/004017
(87) International publication number: WO 2003/083111

(57) **Abstract**

In order to achieve an object of providing a nucleic acid library and a protein library capable of supplying plural species of proteins correlated with the nucleic acid coding therefor at once, and analyzing immediately after the supply the plural species of protein in parallel, the present invention provides a nucleic acid library comprising not less than two species of library units present in a state in which they are separated from one another, wherein each library unit comprises a nucleic acid that can express not less than one species of protein in an *in vitro* transcription/translation system or an *in vitro* translation system, and a first protein trapper that is set contiguously to the nucleic acid, each library unit is set on the surface of a solid support, and the nucleic acid contained in each library unit is immobilized on the surface of the solid support where each library unit is set, and a protein library obtainable by subjecting at once not less than two species of library units possessed by the nucleic acid library to an *in vitro* transcription/translation system or an *in vitro* translation system to express at once the nucleic acids contained in the library units.

## Description

### TECHNICAL FIELD

The present invention relates to a nucleic acid library (a group of nucleic acids) comprising a plurality of nucleic acids that are immobilized on a solid support and a protein library (a group of proteins) comprising a plurality of proteins that are displayed on a solid support.

### BACKGROUND ART

In proteomics, in regard to plural species of protein, performing diverse analyses on the structure, function and the like thereof in parallel (that is to say, high throughput analysis) is necessary. For performing analyses of plural species of proteins in parallel, usefulness of protein arrays (protein chip) wherein plural species of proteins are immobilized on the surface of a solid support is drawing attention. In a protein array, plural species of proteins are immobilized in a given region on the surface of a solid support, and each type of protein can be identified by the position on the solid support. Protein arrays are useful for screening proteins that can bind to a target substance (for instance protein, DNA and the like) from among plural species of protein.

### DISCLOSURE OF THE INVENTION

However, in a conventional protein array, non-efficient spotting of proteins onto the surface of a solid support is necessary, while the proteins that are immobilized on the surface of the solid support can easily be denatured or inactivated when dried. Conservation or utilization of conventional protein arrays over a long period of time was difficult owing to the difficulty to regenerate a protein that is immobilized on the surface of a solid support once the protein is denatured or inactivated.

In addition, in assay systems that use a conventional protein array, analyses can be performed only once the supplied protein has been immobilized on the surface of a solid support, such that an analysis cannot start immediately after a protein has been supplied, requiring large amounts of time and effort from the supply of the protein to the beginning of the analyses. That is to say, as the supply of proteins and the protein assay system were distinct and independent technologies, it is difficult to proceed effectively with protein analysis.

Meanwhile, development of *in vitro* transcription/translation systems and the like that use a phage promoter progressed, allowing a target protein to be supplied efficiently in a short time. In addition, the phage display method (Smith, G. P., 1985, Science, 228, 1315-1317), the ribosome display method (Hanes, J. and Pluckthun, A. 1997, Proc. Natl. Acad. Sci. USA, 94, 4937-4942) and the like have been developed, allowing the target protein to be supplied in a state in which it is correlated with the nucleic acid coding therefor. If the target protein (phenotype), which is to be the subject of the analyses, can be supplied in a state in which it is correlated withthenucleicacidcodingtherefor (genotype) , the amino acid sequence of the target protein can be easily identified, which is advantageous in the analyses of the target protein.

In addition, a technique has also been developed to supply the target protein in a state in which it is correlated with the nucleic acid coding therefor, using a DNA having at least a transcription translation start region, a region coding for the target protein and a region coding for an adapter protein (also referred to as a "tag protein" in the present invention), and to which a ligand is bound (Japanese Patent Application Laid-open No. 2001-128690). This technique allows the target protein to be supplied in a state in which it is correlated with the DNA coding therefor by expressing the above-mentioned DNA in a cell-free transcription translation system and synthesizing the target protein as a fusion protein with the adapter protein, and by binding the fusion protein to the DNA via a specific binding between the ligand bound to the DNA and the adapter protein. In addition, analyses of plural species of proteins can be performed in parallel, using the library of protein-DNA coupled molecules created by this technique. However, in this technique, the DNA must be expressed by isolating each species or each molecule in order to prevent cross-contamination of proteins expressed by different species of DNA.

Thus, an object of the present invention is to provide a nucleic acid library, a protein library and a method for making a protein library allowing plural species of proteins to be supplied at once in a state in which they are correlated with the nucleic acids coding therefor, while allowing analyses of plural species of proteins to be performed in parallel immediately after supplying the plural species of protein, and furthermore allowing a denatured or inactivated protein to be regenerated easily. In addition, an object of the present invention is to provide a particle that is useful as a library unit to constitute a nucleic acid library and a protein library.

In order to achieve the aforementioned objects, the present invention provides the following nucleic acid library, protein library, method for making a protein library, kit for making a protein library, and particle.

(1) The nucleic acid library of the present invention is a nucleic acid library comprising not less than two species of library units present in a state in which they are separated from one another, wherein each library unit comprises a nucleic acid that can express not less than one species of protein in an *in vitro* transcription/translation system or an *in vitro* translation system, and a first protein trapper that is set contiguously to the nucleic acid, each library unit is set on the surface of a solid support, and the nucleic acid contained in each library unit is immobilized on the surface of the solid support where each library unit is set.

In the nucleic acid library of the present invention, as different species of library units are separated from one another while a protein trapper (first protein trapper) is set contiguously to a nucleic acid in each library unit, the distance between a nucleic acid and a protein trapper contained in different species of library unit is considerably greater than the distance between a nucleic acid and a protein trapper contained in the same library unit. Therefore, as a protein expressed by a nucleic acid contained in each library unit is trapped preferentially by a protein trapper (first protein trapper) contained in the same library unit rather than by a protein trapper (first protein trapper) contained in a different species of library unit, cross-contamination of a protein expressed in a different species of library unit can be effectively prevented. Therefore, when the two or more species of library units of the nucleic acid library of the present invention are subjected to an *in vitro* transcription/translation system or an *in vitro* translation system, the protein expressed by a nucleic acid that is contained in each library unit is displayed on the solid support, trapped by a protein trapper (first protein trapper) contained in the same library unit. That is to say, according to the nucleic acid library of the present invention, plural species of proteins can be supplied at once in a state in which they are correlated with the nucleic acids coding therefor and in a state in which they are displayed on a selid support, which allows analyses of plural species of proteins to be performed efficiently in parallel.

In addition, the nucleic acid library of the present invention is immediately converted into a protein library by subjecting each library unit to an *in vitro* transcription/translation system or an *in vitro* translation system. That is to say, in the nucleic acid library of the present invention, as the supply of protein and the protein assay system are integrated, analyses of plural species of proteins can be performed immediately after supplying the plural species of proteins.

In addition, in the nucleic acid library of the present invention, even if a protein that is displayed on the solid support has been denatured or inactivated, the protein can be easily regenerated by re-expressing the nucleic acid corresponding to the protein.

In addition, the nucleic acids contained in each library unit of the nucleic acid library of the present invention can be conserved over a relatively long period of time compared to proteins. Therefore, the protein library made from the nucleic acid library of the present invention can be repeatedly used over a long period of time, by conserving it in the state of nucleic acid and regenerating the protein library when needed.

(2) In a preferred aspect of the nucleic acid library as recited in (1), all the library units are set on the surface of a single solid support.

In the nucleic acid library according to the present aspect, as all the library units can be handled at once by handling a single solid support, handling a nucleic acid library is easy.

(3) In a preferred aspect of the nucleic acid library as recited in (2), the position of each library unit on the surface of the solid support is correlated with the species of each library unit.

In the nucleic acid library according to the present aspect, the species of each library unit, the species of nucleic acid contained in each library unit and the species of the protein expressed by the nucleic acid contained in each library unit can be identified based on the position on the solid support of each library unit.

(4) In a preferred aspect of nucleic acid library as recited in (2) or (3), the solid support has a shape that can be wound around an axial member.

When arranging plural species of DNA at high density on a plane, problems exist such that the considerable costs and large amounts of labor and time are required for manufacturing a DNA array, cross-contamination by different species of DNA occurs easily and the like. Thus, an accumulation support has been developed, having a base member with an elongated shape such as a string-shape, a cord-shape or a tape-shape, wherein various substances for detection havingagivenchemicalstructureareimmobilized so as to be aligned along the longitudinal direction, and wherein the chemical structure is correlated with the immobilization position thereof, and a support around which the aforementioned base member is wound; a DNA array with DNAs arranged at high density can be made easily and at low cost while preventing cross-contamination using this accumulation technique, by immobilizing DNAs onto the solid support with an elongated shape such as a string-shape, a cord-shape or a tape-shape in a state in which it is spread (primary accumulation) , then winding this around the axial member (secondary accumulation) (WO 01/69249A1, WO 01/53831A1, WO 02/63300A1).

The nucleic acid library according to the present aspect uses this accumulation technique. That is to say, in the nucleic acid library according to the present aspect, each library unit can be arranged at high density by setting, in a state in which a solid support having a shape that is windable around an axial member (elongated shape such as for instance, cord shape, tape shape and string shape) is spread, each library unit onto the solid support (primary accumulation), then winding this around the axial member (secondary accumulation). Thereafter, by converting the nucleic acid library in which each library unit is arranged at high density into a protein library, a protein array can be made, wherein proteins are arranged at high density. Therefore, automation of a series of operations from making a protein library to analyzing proteins with the protein library can be easily realized by using the nucleic acid library in which a solid support has been wound around an axial member.

In addition, a protein array in which proteins are arranged at high density can be made by converting, in a state in which the solid support having a shape that is windable around an axial member is spread, the nucleic acid library into a protein library, then winding the solid support around the axial member.

(5) In a preferred aspect of the nucleic acid library as recited in (1) , different species of library units are respectively set on the surface of separate solid supports.

In the nucleic acid library according to the present aspect, the nucleic acids contained in different species of libraryunits are respectively immobilized on the surface of separate solid support. As the solid support is a presence that is macro relatively to nucleic acids and protein trappers, the distance between a nucleic acid and a protein trapper contained in different species of library units is considerably larger than the distance between a nucleic acid and a protein trapper contained in the same library unit and at the same time the probability of an encounter between a nucleic acid and a protein trapper contained in different species of library units is maintained at an extremely low level. Therefore, a protein expressed by a nucleic acid contained in a library unit that is set on the surface of each solid support is trapped preferentially by a protein trapper (first protein trapper) contained in a library unit that is set on the surface of the same solid support, allowing cross-contamination of a protein expressed in a library unit that is set on the surface of a separate solid support to be effectively prevented.

In addition, in the nucleic acid library according to the present aspect, as the degree of freedom for combining the library units increases, different species of protein can be freely combined and analyzed.

(6) In a preferred aspect of the nucleic acid library as recited in (5), the solid supports onto which different species of library units are set are mutually distinct and identifiable.

In the nucleic acid library according to the present aspect, the species of each library unit, the species of a nucleic acid contained in each library unit and the species of a protein that is expressed by a nucleic acid contained in each library unit can be identified by identifying the solid support onto which each library unit is set.

(7) In a preferred aspect of the nucleic acid library as recited in (5) or (6), the solid support is a particle.

In the nucleic acid library according to the present aspect, a particle onto which each library unit is set can be dispersed in a liquid (see (9) refer in the following) .

(8) In a preferred aspect of the nucleic acid library as recited in (7), the particle possesses magnetism.

In the nucleic acid library according to the present aspect, operational performance of the solid support onto which each library unit is set increases, allowing automation of the creation of the protein library using the nucleic acid library of the present invention and automation of protein analysis using the protein library of the present invention to be realized easily.

(9) In a preferred aspect of the nucleic acid library as recited in (7) or (8), the particle is dispersed in a liquid.

In the nucleic acid library according to the present aspect, reactivity in each library unit is increased by dispersing in a liquid particles onto which each library unit is set, such that when subjecting the nucleic acid library according to the present aspect to an *in vitro* transcription/translation system, the transcription reaction and translation reaction in each library unit can proceed rapidly. As the particles that are dispersed in the liquid can be collected using a magnet if the particles possess magnetism, allowing the particles to be easily separated from the liquid, it is preferred that the particles to be dispersed in the liquid have magnetism.

Note that a state in which a solid support onto which each library unit is set is dispersed in a liquid is also included in the nucleic acid library of the present invention. In addition, in a state in which a particle onto which each library unit is set is dispersed in a liquid, the distance between a nucleic acid and a protein trapper contained in different species of library units is also considerably larger than the distance between a nucleic acid and a protein trapper contained in the same library unit and at the same time the probability of an encounter between a nucleic acid and a protein trapper contained in different species of library units is also maintained at an extremely low level. Therefore, a protein expressed by a nucleic acid contained in a library unit that is set on the surface of each particle is trapped preferentially by a protein trapper (first protein trapper) contained in a library unit that is set on the surface of the same particle, allowing cross-contamination of a protein expressed in a library unit that is set on the surface of a separate particle to be effectively prevented.

(10) In a preferred aspect of the nucleic acid library as recited in (9), a particle onto which no nucleic acid that can express a protein in an *in vitro* transcription/translation system or an *in vitro* translation system is immobilized is mixed in the liquid.

In the nucleic acid library according to the present aspect, the distance between the particles onto which each library unit is set is increased by the presence between the particles onto which each library unit is set, of particles onto which no nucleic acid that can express a protein in an *in vitro* transcription/translation system or an in vitro translation system is immobilized. In this way, the protein trapper (first protein trapper) contained in a library unit that is set on the surface of each particle, can trap preferentially a protein expressed by a library unit that is set on the surface of the same particle over a protein expressed by a library unit that is set on the surface of another particle, allowing cross-contamination of a protein expressed in a different species of library unit to be effectively prevented. In addition, in the nucleic acid library according to the present aspect, the problem of decrease in the operational performance of the particles, which arise when the concentration of particles onto which each library unit is set becomes low (for instance, when the concentration of the particles is lowered to increase the distance between the particles), can be eliminated. Note that, as no nucleic acid that can express a protein in an in vitro transcription/translation system or an in vitro translation system is immobilized on the particle to be mixed, no protein is expressed from the particle, such that no cross-contamination of protein can arises from the particle to each library unit.

(11) In a preferred aspect of the nucleic acid library as recited in (9) or (10), a second protein trapper or an RNA polymerase binder is mixed in the liquid.

In the nucleic acid library according to the present aspect, even if a protein expressed from a library unit set on the surface of each particle is not trapped by a protein trapper (first protein trapper) contained in the same library unit and is dispersed into the liquid, trapping of the protein by a protein trapper contained in a library unit that is set on the surface of another particle can be prevented by trapping the protein with the protein trapper (second protein trapper) present in the liquid.

In addition, as the transcription reaction in the library unit containing DNA as nucleic acid is inhibited by binding of the RNA polymerase contained in the *in vitro* transcription/translation system RNA to the polymerase binder, expression from the library unit and dispersion into the liquid of excessive mRNA can be prevented effectively.

Therefore, in the nucleic acid library according to the present aspect, cross-contamination of a protein expressed in a different species of library unit can be effectively prevented.

(12) In a preferred aspect of the nucleic acid library as recited in any of (1) to (11), the solid support is porous.

In the nucleic acid library according to the present aspect, a library unit can be set on the internal surface of a pore of the solid support. As the fluidity of a liquid (for instance a solution of an *in vitro* transcription/translation system or an *in vitro* translation system) inside the pores of the solid support is low, protein expressed from a nucleic acid contained in the library unit that is set on the internal surface of a pore of the solid support is not easily released from the interior of the pore. Therefore, a protein expressed from a nucleic acid is trapped by a protein trapper (first protein trapper) contained in the same library unit with certainty, allowing cross-contamination of a protein expressed in a different species of library unit to be effectively prevented.

(13) In a preferred aspect of the nucleic acid library as recited in (12), the solid support is a fiber or an aggregate thereof.

In the nucleic acid library according to the present aspect, since the solid support is a fiber or an aggregate thereof, the solid support becomes porous. If the solid support is an aggregate of fibers (for instance twisted fibers), many pores are present in the solid support.

(14) In a preferred aspect of the nucleic acid library as recited in any of (1) to (13), in any one or more of the library units, one end of the nucleic acid is immobilized onto the solid support and the first protein trapper is set at the other end of the nucleic acid.

In the nucleic acid library according the present aspect, a protein trapper (first protein trapper) is set contiguously to a nucleic acid by setting the protein trapper (first protein trapper) at an end of the nucleic acid.

(15) In a preferred aspect of the nucleic acid library as recited in any of (1) to (14), the first protein trapper is set so as to surround the nucleic acid in any one or more library units.

In the nucleic acid library according the present aspect, a protein expressed from a nucleic acid is trapped by a protein trapper (first protein trapper) contained in the same library unit with certainty by setting a protein trapper (first protein trapper) so as to surround the nucleic acid, allowing cross-contamination of a protein expressed in a different species of library unit to be effectively prevented. Since the more the number of library units in which the first protein trapper is set so as to surround a nucleic acid increases, the more cross-contamination of a protein expressed in a different species of library unit can be effectively prevented, it is preferred that the number of library units in which the first protein trapper is set so as to surround a nucleic acid is as many as possible, setting the first protein trapper so as to surround a nucleic acid in every library unit being most preferred.

(16) In a preferred aspect of the nucleic acid library as recited in any of (1) to (15), the protein that can be expressed by the nucleic acid in any one or more library units is a fusion protein between a target protein and a tag protein that can bind to the first protein trapper.

In the nucleic acid library according the present aspect, binding between a protein that is expressed by a nucleic acid and a protein trapper (first protein trapper) contained in the same library unit as the nucleic acid is ensured. Note that a "target protein" means a protein that is to be the subject of the analyses.

If fusion proteins are to be expressed from a plurality of library units, it is preferred that these fusion proteins bind to the first protein trappers so as to be in the same orientation. For any fusion protein, the reactivity of the fusion protein can be unified and optimized by having the same orientation.

(17) In a preferred aspect of the nucleic acid library as recited in (16), the tag protein can bind to the second protein trapper.

In the nucleic acid library according the present aspect, even if a protein expressed from a nucleic acid is not trapped by a protein trapper (first protein trapper) contained in the same library unit as the nucleic acid and is dispersed into the liquid, the protein can be trapped with certainty by the protein trapper (second protein trapper) present in the liquid.

(18) In a preferred aspect of the nucleic acid library as recited in any of (1) to (17), in the library unit containing a DNA as the nucleic acid, an mRNA trapper is set contiguously to the DNA.

In the nucleic acid library according the present aspect, in a library unit containing a DNA as the nucleic acid, the mRNA generated by the transcription of the DNA is trapped by an mRNA trapper contained in the same library unit. Therefore, the mRNA arising from the library unit containing a DNA as the nucleic acid when the nucleic acid library according the present aspect is subjected to an in vitro transcription/translation system can be prevented from being dispersed into the solution for the in vitro transcription/translation system. In this way, a protein generated by the translation of an mRNA is prevented from being trapped by a protein trapper contained in another library unit, and cross-contamination of a protein expressed in a different species of library unit can be effectively prevented.

(19) The protein library of the present invention is a protein library obtainable by subjecting at once not less than two species of library units possessed by the nucleic acid library as recited in any of (1) to (18) to an in vitro transcription/translation system or an in vitro translation system to express at once the nucleic acids contained in the library units.

Since cross-contamination of a protein expressed in a different species of library unit is prevented even if not less than two species of library units possessed by the nucleic acid library are subjected at once to an *in vitro* transcription/translation system or an *in vitro* translation system, plural species of proteins are displayed at once on the solid support in a state in which they are correlated with the nucleic acids coding therefor. Therefore, according to the protein library of the present invention, analyses of plural species of proteins can be performed effectively in parallel.

In addition, as the protein library of the present invention is made immediately by expressing the nucleic acid contained in each library unit of the nucleic acid library of the present invention, analyses of plural species of proteins can be performed immediately after supplying the plural species of protein.

In addition, in the protein library of the present invention, even if the protein displayed on the solid support has been denatured or inactivated, the protein can easily be regenerated by re-expressing the nucleic acid corresponding to the protein.

In addition, the protein library of the present invention can be used repeatedly over a long period of time by conserving it in the state of a nucleic acid library, and regenerating the protein library when needed.

(20) The method for creating protein library of the present invention is a method for making a protein library, comprising subjecting at once not less than two species of library units possessed by the nucleic acid library as recited in ay of (1) to (18) to an *in vitro* transcription/translation system or an *in vitro* translation system to express at once the nucleic acids contained in the library units.

In the method for making a protein library of the present invention, a protein library of the present invention can be made in a state in which cross-contamination of a protein expressed in a different species of library unit is prevented.

(21) In a preferred aspect of the method for creating protein library as recited in (20), a second protein trapper is mixed in the *in vitro* transcription/translation system or the *in vitro* translation system.

In the method for making a protein library according to the present aspect, even if a protein expressed from each library unit is not trapped by a protein trapper (first protein trapper) contained in the same library unit and is dispersed into the solution of the *in vitro* transcription/translation system or the *in vitro* translation system, trapping of the protein by a protein trapper (first protein trapper) contained in another library unit can be prevented by trapping the protein with a protein trapper (second protein trapper) present in the solution. Therefore, a protein library of the present invention can be made in a state in which cross-contamination of a protein expressed in a different species of library unit is effectively prevented.

(22) In a preferred aspect of the method for creating protein library as recited in (20) or (21), the nucleic acids are expressed at once while the transcription reaction in the *in vitro* transcription/translation system is being inhibited.

In the method for making protein library according to the present aspect, as the transcription reaction in the *in vitro* transcription/translation system is being inhibited, excessive expression of mRNA from a library unit containing DNA as nucleic acid, and dispersion of the mRNA into the solution of the *in vitro* transcription/translation system can be prevented. Therefore, a protein library of the present invention can be made in a state in which cross-contamination of a protein expressed in a different species of library unit is effectively prevented.

(23) In a preferred aspect of the method for creating protein library as recited in (22), an RNA polymerase binder is mixed in the *in vitro* transcription/translation system to inhibit the transcription reaction in the *in vitro* transcription/translation system.

In the method for making a protein library according to the present aspect, as the transcription reaction in the library unit containing DNA as nucleic acid is inhibited by binding of RNA polymerase contained in the *in vitro* transcription/translation system to an RNA polymerase binder, expression of excessive mRNA from the library unit can be effectively prevented.

(24) In a preferred aspect of the method for creating protein library as recited in (22) or (23), the transcription reaction in the *in vitro* transcription/translation system is inhibited by adjusting the temperature of the *in vitro* transcription/translation system.

In the method for creating protein library according to the present aspect, as the transcription reaction in the library unit containing DNA as nucleic acid is inhibited by adjusting the temperature of the in vitro transcription/translation system and decreasing the reactivity of RNA polymerase contained in the *in vitro* transcription/translation system, expressionof excessive mRNA from the library unit can be effectively prevented.

Note that a possibility exists that not only the transcription reaction but also the translation reaction will be inhibited by adjusting the temperature of the in vitro transcription/translation system; in such a case, the inhibition of the translation reaction can be prevented by pre-including in the in vitro transcription/translation system larger amounts of factors that are involved in the translation reaction.

(25) The kit formaking a protein library of the present invention is a kit for making a protein library, comprising the nucleic acid library as recited in any of (1) to (18) and a second protein trapper or an RNA polymerase binder.

According to the kit for making a protein library of the present invention, a protein library of the present invention can be made in a state in which cross-contamination of a protein expressed in a different species of library unit is effectively prevented, by the aforementioned effective action of the second protein trapper or of the RNA polymerase binder. The kit for making a protein library of the present invention may contain, in addition to a nucleic acid library, a second protein trapper and an RNA polymerase binder, any elements that are required to make the protein library.

(26) The particle of the present invention is a particle on the surface of which nucleic acids that can express not less than one species of protein in an *in vitro* transcription/translation system or an *in vitro* translation system are immobilized at high density, wherein one end of each nucleic acid is immobilized on the surface of the particle and a protein trapper is set at the other end of each nucleic acid.

As a barrier of protein trapper is formed contiguously to the nucleic acid on the particle on the outside of the particle of the present invention, the nucleic acid on the particle being in a state in which it is surrounded by the protein trapper, even if a plurality of particles are supplied at once to an *in vitro* transcription/translation system or an *in vitro* translation system, the protein expressed on each particle is trapped by a protein trapper on the same particle, allowing cross-contamination of protein expressed on each particle to be effectively prevented.Therefore,theparticleofthe presentinvention is useful as a library unit for constructing the nucleic acid library and the protein library of the present invention.

### BRIEF DESCRIPTION OF THE DRAWINGS

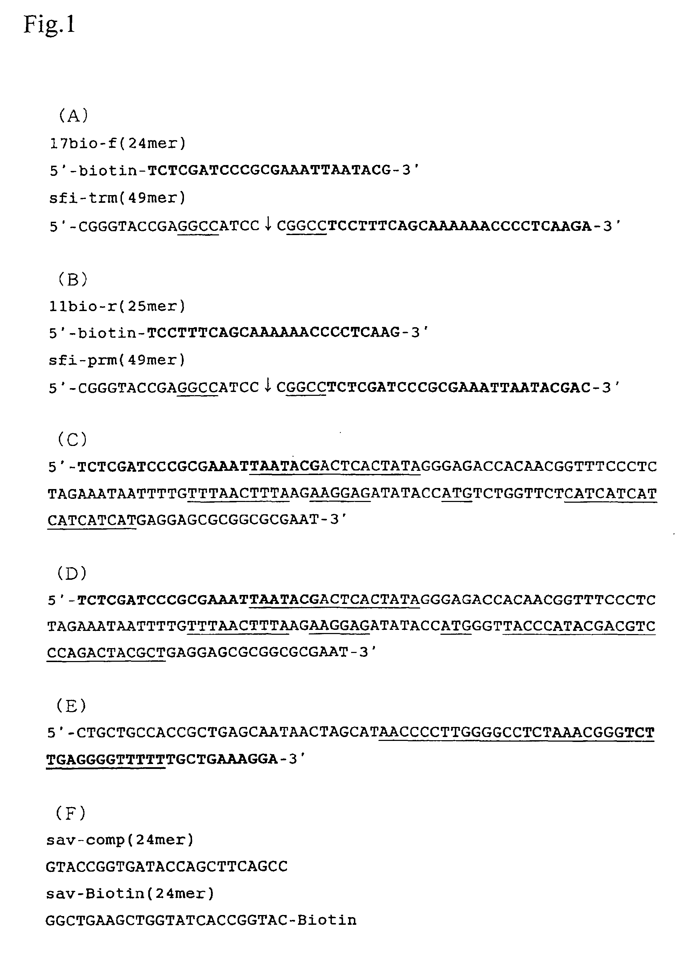

FIG. 1 (A) shows, in regard to the His-labeled and HA-labeled avidin genes, the base sequences of PCR primers for introducing biotin on the 5' side and an SfiI site on the 3' side, FIG. 1 (B) shows, in regard to the His-labeled and HA-labeled avidin genes, the base sequences of PCR primers for introducing an SfiI site on the 5' side and biotin on the 3' side, FIG. 1 (C) shows a structure that is upstream of the start codon of the avidin gene portion in the His-labeled avidin gene, FIG. 1 (D) shows a structure that is upstream of the start codon of the avidin gene portion in the HA-labeled avidin gene, FIG. 1 (E) shows a structure that is downstream of the termination codon of the avidin gene portion in the His-labeled avidin gene and the HA-labeled avidin gene, FIG. 1 (F) shows structures of the oligo DNAs that were used in the preparation of the double-stranded DNA that is immobilized on an sav bead, having a particle diameter of 0.7µm.
FIG. 2 (A) shows the base sequences of an adapter, FIG. 2 (B) schematically shows how the biotinylated adapter is ligated to the SfiI site.
FIG. 3 shows the results after beads on which different species of DNA fragment have been immobilized were subjected to an *in vitro* transcription/translation system with mixing or without mixing (2 hours or 4 hours), and the beads were separated by flow cytometry.
FIG. 4 shows the results after beads on which different species of DNA fragment have been immobilized were subjected to an *in vitro* transcription/translation system with mixing or without mixing (1 hour at 30°C or 1 hour 45 minutes at 37°C), and the beads were separated by flow cytometry.
FIG. 5 shows the results after beads on which different species of DNA fragment have been immobilized were subjected to an in vitro transcription/translation system (1 hour 15 minutes at 30°C or 1 hour 45 minutes at 37°C) with mixing or without mixing, in the presence or in the absence of a DNA fragment (17mer, 60mer) having a T7 promoter sequence, and the beads were separated by flow cytometry.
FIG. 6 shows the locations where the primers that were used in the PCRs (first time and second time) for determining the species of the DNA fragment that is immobilized on the surface of the bead hybridizes.
FIG. 7 is a perspective view showing one mode of the nucleic acid library, in which a cord-shaped solid support is wound around a cylindrical axial member.

### BEST MODE FOR CARRYING OUT THE INVENTION

In the following, the present invention will be explained in detail.

The nucleic acid library of the present invention comprises not less than two species of library units. The differentiation of library units species is determined by the differentiation of the species of the proteins that are expressed form the library units. That is to say, library units that are in a relationship in which they express not less than one species of different proteins are different species of library units, and library units that are in other relationships are the same species of library unit. Therefore, "comprises not less than two species of library units" means to comprises a plurality of library units that can respectively express at least one species of different protein. The nucleic acid library of the present invention may comprise a plurality of the same species of library units as long as it comprises not less than two species of library units. Note that, this includes obviously library units that are in a relationship in which only the same species of protein is expressed in the same species of library unit, and also library units that are in a relationship in which one expresses different species of protein, but the other does not express different species of protein (for instance in case where one library unit expresses two species of proteins, e.g., protein A and protein B, and the other library unit expresses one species of protein, e.g., protein A).

In the nucleic acid library of the present invention, different species of library units are present in a state in which they are separated from one another. The distance between different species of library units is not limited in particular as long as a protein expressed from a nucleic acid contained in each library unit may be preferentially trapped by a protein trapper contained in the same library unit rather than by a protein trapper contained in a different species of library unit, the distance between a protein trapper and a nucleic acid contained in a different species of library unit being set to be considerably greater than the distance between a protein trapper and a nucleic acid contained in the same library unit.

In case the nucleic acid library of the present invention comprises a plurality of the same species of library units, the same species of library units may be present in a state in which they are separated from one another, or in a state in which they are contiguous to one another. In case the same species of library units are present in a state in which they are contiguous, although a protein expressed in each library unit is sometimes trapped by a protein trapper contained in a different library unit, the problem of cross-contamination does not arise if it is the same species of library unit.

In the nucleic acid library of the present invention, each library unit is set on the surface of a solid support. Since there are cases where the nucleic acid library of the present invention is used in a water based medium (for instance, in case the nucleic acid library of the present invention is subjected to a cell-free transcription/translation system, which was prepared from a cell extract), it is preferred that the solid support onto which each library unit is set be insoluble with respect to water. Examples of materials for such a solid support include glass, silicon, ceramics, water-insoluble polymers (for instance, synthetic resins such as polystyrene and other polystyrene resins, polymethylmethacylate and other acrylic resins (methacrylic resins), polyamide resins, polyethylene terephthalate and other polyesters, polycarbonate and the like; polysaccharides such as agarose, dextran, cellulose and the like; proteins such as gelatin, collagen, casein and the like) and the like. The surface of the solid support may be planar or curved, or may have asperities.

The solid support may be porous or non-porous; however it is preferred that it is porous. Examples of porous solid supports include, for instance, fiber or an aggregate thereof (for instance twisted fibers). In case the solid support is porous, the library unit can be set on the internal surface of the pores. As the fluidity of a liquid (for instance a solution of an *in vitro* transcription/translation system or an *in vitro* translation system) inside the pores of the solid support is low, protein expressed from a nucleic acid contained in the library unit that is set on the internal surface of a pore of the solid support is not easily released from the interior of the pore. Therefore, the protein can be trapped by a protein trapper (first protein trapper) contained in the same library unit with certainty, allowing cross-contamination of a protein expressed in a different species of library unit to be effectively prevented.

The shape of the solid support is not restricted in particular and can take any shape, for instance, a plate-shape, a particle (bead)-shape, a rod-shape, a cord-shape, a tape-shape, a string-shape and the like; however it is preferred that the solid support has a shape that is windable around an axial member or a particle (bead)-shape. Examples of shapes that are windable around an axial member include elongated shapes such as a cord-shape, a tape-shape and a string shape. The shape or the structure of the axial member is not limited in particular as long as it may serve as the center of the object to wind, and examples of members include, for instance, a rod-shape, a cylinder-shape, a tube-shape, a prism-shape, a hollow prism shape and the like. A nucleic acid library in which each library unit is arranged at high density and a protein array in which proteins are arranged at high density can be made by using a solid support having a shape that is windable around an axial member. In addition, automation of a series of operations from making a protein library to analyzing proteins using the protein library can be easily realized by using the nucleic acid library in which a solid support has been wound around an axial member.

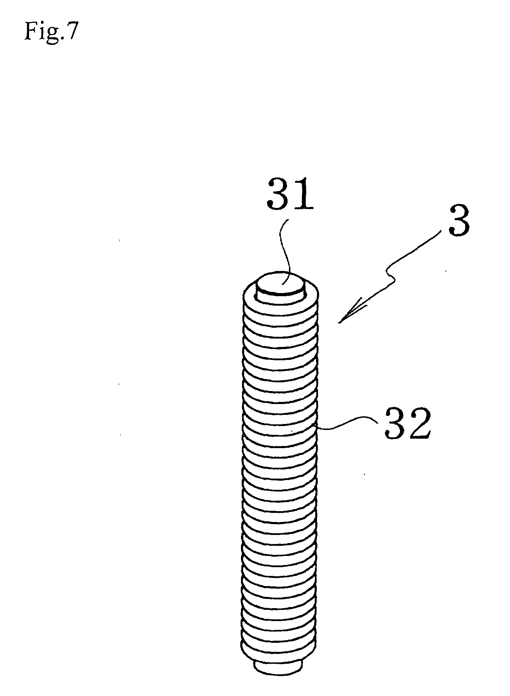

A nucleic acid library 3 in which a cord-shaped solid support 32 that is wound around a cylinder-shaped axial member 31 is shown in FIG. 7 as one embodiment of a nucleic acid library in which the solid support has a shape that is windable around an axial member. A plurality of different library units are set on the surface of the cord-shaped solid support 32.

The solid support may have magnetism, and in particular, it is preferred that is has magnetism when the solid support is particle-shaped. When a magnetic particle is used as the solid support, operational performance is improved, allowing automation of making the protein library using the nucleic acid library of the present invention and automation of analyzing protein using the protein library of the present invention to be realized easily. Note that the shape of a "particle" is not restricted in particular, and is, for instance, globular. In addition, the size of a particle is also not restricted in particular; however, a particle diameter of 0.2 to 30µm is preferred.

In the nucleic acid library of the present invention, the "surface" of a solid support onto which each library unit is to be set means a surface that may be in contact with a liquid (for instance a solution of an *in vitro* transcription/translation system or an *in vitro* translation system), including obviously the outer surface (external surface) of the solid support, but also the inner surface (internal surface) of the solid support into which a liquidmay infiltrate (for instance, the internal surface of the pores that the solid support possesses).

The nucleic acid library of the present invention is a group of plural library units. The mode of grouping library units is not restricted in particular, such that all the library units may be grouped in a state in which they are set together on the surface of a single solid support (for instance, a solid support having a shape that is windable around an axial member), or they may be grouped in a state in which each library unit is set separately on the surface of plural solid supports. In addition, each library unit may be set separately on the surface of plural solid supports (for instance particles), and grouped in a state in which they are dispersed in a liquid (for instance, in a state in which they are dispersed in a given volume of liquid contained in a container).

In case each library unit is set separately on the surface of plural solid supports, the number of library units to be set on each solid support may be one or plural, and the species of library unit to be set on each solid support may be identical or different.

In case different species of library units are set on the surface of the same solid support, the different species of library units are set so as to be separated from one another. In addition, in case different species of library units are set respectively on separate solid supports, since a solid support is a presence that is macroscopic relatively to nucleic acids and protein trappers, the different species of library units are separated from one another.

In the nucleic acid library of the present invention, it is preferred that all the library units be set on the surface of a single solid support, or, that different species of library units are respectively set on the surface of separate solid supports.

In case different species of library units are set respectively on the surface of separate solid support, if plural identical species of library units are present, the identical species of library units may be set on the surface of the same solid support, or may be set on the surface of separate solid supports.

In the nucleic acid library of the present invention, it is preferred that each library unit is grouped in a state in which the species thereof is identifiable. As the species of the nucleic acid contained in each library unit and the species of a protein expressed from the nucleic acid contained in each library unit can be also be identified by identifying the species of each library unit, protein analyses can be performed effectively.

In case all the library units are set on the surface of a single solid support, the species of each library unit can be identified based on the location of each library unit on the solid support, for instance by correlating the location of each library unit on the solid support with the species of each library.

In addition, in case different species of library units are set respectively on the surface of separate solid supports, the species of each library can be identified based on the species of each solid support onto which the library unit has been set, for instance, by using solid supports that are mutually distinct and identifiable.

In addition, the species of a library unit can also be identified based on the label of the library unit per se, by labeling a constitutive element of different species of library units with different labeling substances. Examples of constitutive elements of the library unit that can be labeled with a labeling substance include, for instance, nucleic acid that can express not less than one species of protein in an in vitrotranscription/translation system or an *in vitro* translation system, first protein trapper, mRNA trapper, element for immobilizing the first protein trapper onto the solid support (for instance nucleic acid that can not express a protein in an *in vitro* transcription/translation system or an *in vitro* translation system) and the like.

Identification of the solid support is possible, for instance, by labeling the solid supports onto which different species of library units are set with different labeling substances. Specific examples of labeling substances include fluorescent substances such as fluorescent dyes (for instance, in addition to Marine Blue, Cascade Blue, Cascade Yellow, Fluorescein, Rhodamine, Phycoerythrin, CyChrome, PerCP, Texas Red, Allophycocyanin, PharRed and the like, dyes of the Cy series such as Cy2, Cy3, Cy3.5, Cy5 and Cy7, dyes of the Alexa series such as Alexa-488, Alexa-532, Alexa-546, Alexa-633, Alexa-680, and dyes of the BODIPY series such as BODIPY FL and BODIPY TR), radioactive substance such as radioactive isotopes (for instance, ³H, ¹⁴C, ³²P, ³³P, ³⁵S and ¹²⁵I) and the like. When using a fluorescent dye as a labeling substance, a variety of labeling becomes possible by combining the species and the content of the fluorescent dye. Labeling of a solid support by a fluorescent dye can be performed, for instance, by reacting a fluorescent dye having an active ester onto a solid support on the surface of which an amino group has been introduced beforehand, or, by reacting, in the presence of carbodiimides such as 1-ethyl-3-(3-dimethylaminopropyl)-3-ethylcarbodiimide hydrochloride (EDC) or the like, a fluorescent dye having a functional group allowing a binding reaction with a carboxyl group (for instance an amino group) or a fluorescent dye having a functional group allowing a binding reaction with an amino group (for instance a carboxyl group), onto a solid support on the surface of which a carboxyl group or an amino group has been introduced beforehand. In addition, in case the solid support is a particle, labeling of the particle by a fluorescent dye is possible, for instance, by having a fluorescent dye added in the reaction solution when synthesizing the particle via a polymerization reaction, or, by adding, immediately after the end of the polymerization reaction of a radical polymerization and while radicals are still surviving, a fluorescent dye having reactivity with the radicals.

In addition, identification of the solid support becomes possible also by differentiating the shapes and the sizes of the solid supports onto which different species of library units are set. For instance, in case the solid supports are particle (bead)-shaped, identification of the solid supports becomes possible by differentiating the particle diameters thereof. Identification of particles with different particle diameters can be performed for instance by using flow cytometry or the like.

In addition, identification of the solid support becomes possible also by differentiating the physical characteristics of the solid supports onto which different species of library units are set. Identification of the solid support becomes possible for instance based on the difference in magnetization when a magnet is approached.

Each library unit comprises a nucleic acid that can express not less than one species of protein in an *in vitro* transcription/translation system or an *in vitro* translation system and a first protein trapper that is set contiguously to the nucleic acid, the nucleic acid contained in each library unit being immobilized on the surface of the solid support onto which each library unit has been set.

The nucleotides that constitute a nucleic acid contained in each library unit may be deoxyribonucleotides or ribonucleotides. That is to say, the nucleic acid contained in each library unit may be any of a DNA and an RNA, DNA and RNA being contained in the "nucleic acid that can express not less than one species of protein in an *in vitro* transcription/translation system" and RNA being contained in the "nucleic acid that can express not less than one species of protein in an *in vitro* translation system". In addition, the base length and the base sequence of the nucleic acid are not restricted in particular and are selected suitably in such a way that the target protein can be expressed. In case the nucleic acid is DNA, it may be immobilized in the double stranded state, or it may be immobilized in the single stranded state; however it is preferred that it is immobilized in the double stranded state. The reason is that transcription of DNA by a polymerase is performed effectively with a double stranded DNA as a substrate.

The number of nucleic acid contained in each library unit is not restricted in particular, and a plurality of nucleic acids (a nucleic acid group) may be contained. In case a plurality of nucleic acids is contained in each library unit, the species of protein that each nucleic acid can express may be identical or different. Since protein analyses can be efficiently performed if one species of protein that is to be the subject of analysis is expressed from each library unit, it is preferred that the proteins that are expressed from each nucleic acid contained in the same library unit be of the same species; however, for instance, in case the protein that is to be the subject of analysis comprises several species of different subunits, it is preferred to include the nucleic acids that express the respective subunit in the same library unit.

In addition, in case a plurality of nucleic acids that can express the same species of protein are contained in each library unit, the structure of each nucleic acid may be identical or different. For instance, nucleic acids with the same open reading frame but different transcription control regions and translation control regions may be contained.

It is preferred that the nucleic acid contained in each library unit be immobilized in such a way that the nucleic acid can not secede from the solid support when the nucleic acid library of the present invention is used in a water based medium. Examples of such methods for immobilizing nucleic acids include, for instance, method for covalently coupling to a functional group on the surface of the solid support (Vera Land, Ruth Schmid, David Rickwood, Erik Hornes (1988). Nucleic Acids Res. 16 (22), 10861), method using biotin-avidin system (Shao-Ochie Huang, Harold Swerclow, and Karin D. Caldwell (1994). Analytical Biochemistry 222, 441-119) and the like. In the former method, examples of functional groups on the surface of the solid support include a carboxyl group, an amino group, a hydroxyl group and the like. For instance, in case a carboxyl group has been formed on the surface of the solid support, the carboxyl group can be activated with carbodiimides such as 1-ethyl-3-(3-dimethylaminopropyl)-3-ethylcarbodiimide hydrochloride (EDC) , and then be coupled to an amino alkyl group introduced in the nucleic acid. In addition, in case an amino group has been formed on the surface of the solid support, the amino group can be converted into a carboxyl group using a cyclic acid anhydride such as succinic anhydride, and coupled to an amino alkyl group that had been introduced in the nucleic acid beforehand, or coupled to the phosphate at the end of the nucleic acid. In the latter method, for instance, a biotinylated nucleic acid can be obtained by performing PCR using primers whose 5' end has been biotinylated beforehand, and immobilize this nucleic acid onto a solid support whose surface has been coated with avidin (or streptavidin).

In each library unit, any portion of the nucleic acid may be immobilized on the solid support; however, it is preferred that the 3' end or 5' end of the nucleic acid be immobilized on the solid support. In this way, proteins can be efficiently expressed from the nucleic acid contained in each library unit.

In each library unit, the "surface" of a solid support onto which a nucleic acid is immobilized means a surface thatmaybe in contact with a liquid (for instance a solution of an *in vitro* transcription/translation system or an *in vitro* translation system), including obviously the outer surface (external surface) of the solid support, but also the inner surface (internal surface) of the solid support into which a liquid may infiltrate (for instance, the internal surface of the pores that the solid support possesses).

The nucleic acid contained in each library unit has a structure that can express not less than one protein in an *in vitro* transcription/translation system or an *in vitro* translation system. The "*in vitro* transcription/translation system" comprises an *in vitro* transcription system that can perform *in vitro* the transcription from a nucleic acid to an mRNA, and an *in vitro* translation system that can perform the translation *in vitro* from an mRNA to a protein, the *in vitro* transcription/translation system containing all the elements that are required for transcription and translation (for instance, RNA polymerase, ribosome, tRNA and the like). Specific examples of *in vitro* transcription/translation systems include cell-free transcription/translation systems prepared from extracts of eucaryotic cells and prokaryotic cells, specific examples of cell-free transcription/translation systems include cell-free transcription/translation systems prepared from cell extracts of Escherichia coli (for instance Escherichia coli S30), wheat germ, rabbit reticulocyte, mouse L-cell, Ehrlich ascites carcinoma cell, HeLa cell, CHO cell, budding yeast and the like.

The structure of the nucleic acid contained in each library unit is not restricted in particular so long as it can express not less than one protein in an *in vitro* transcription/translation system or an *in vitro* translation system. Examples of nucleic acids that can express not less than one species of protein in an *in vitro* transcription/translation system include, for instance, DNA comprising a transcription control region, a translation control region, an open reading frame (ORF) coding for a target protein and a termination codon being set at the 3' side of the open reading frame. Examples of nucleic acids that can express not less than one species of protein in an in vitro translation system include, for instance, RNA having the same structure as the DNA that can express not less than one species of protein in an in vitro transcription/translation system, except the point that thymine (T) is uracil (U). However, in case of RNA, a transcription control region is unnecessary.

Specific examples of transcription control regions include a promoter, a terminator, an enhancer and the like, specific examples of translation control regions include the kozak sequence, the Shine-Dalgarno (SD) sequence and the like. The transcription control region and the translation control region can be of any species so long as they respectively allow for a transcription from DNA to mRNA and for a translation from mRNA to protein, an can be suitably selected according to the species of the *in* vitro transcription system and the like. In addition, the transcription control region and the translation control region may exist as separate regions or may exist by being overlapped.

The number of open reading frames is not restricted in particular, one open reading frame may exist by itself so that one species of protein is expressed, or a plurality of same species of open reading frames may exist in separated states. In addition, not less than two species of open reading frames may exist in separated states so that not less than two species of proteins are expressed. In addition, not less than two species of open reading frames may exist in a linked state, such that a fusion protein is expressed. Note that a fusion protein is included in "one species of protein".

Examples of specific structures of a nucleic acid contained in each library unit include, for instance, a structure having a promoter and an SD sequence on the 5' side of the open reading frame and a termination codon and a terminator on the 3' side of the open reading frame.

A protein trapper (first protein trapper) contained in each library unit may be any entity as long as it can trapaprotein. It is preferred that a protein trapper (first protein trapper) contained in each library unit can specifically trap a protein expressed from a nucleic acid contained in each library unit; however, in such case, as a different protein trapper becomes necessary for each species of library unit, construction of the nucleic acid library of the present invention becomes difficult. Therefore, generally, a protein trapper capable of nonspecifically trapping any protein is used as the first protein trapper. For instance, a protein trapper that can nonspecifically trap any protein by chemical coupling, electric coupling or physical coupling can be used as a first protein trapper. In addition, a physical obstacle that limits the movement of the protein from the library unit in the direction of the division (that is to say, can retain the protein in the vicinity of the library unit) can also be used as a first protein trapper. Note that the species of the first protein trapper and the scheme of protein trapping by the first protein trapper may be common or different among the respective library units.

For instance, in case a protein expressed from a nucleic acid contained in a library unit is a fusion protein between a target protein that is to be a subject of analyses and a tag protein that can chemically be coupled to a first protein trapper, the first protein trapper can trap the expressed protein (fusion protein) via chemical coupling. Examples of combinations of tag protein/protein trapper include, for instance, biotin binding protein such as avidin andstreptavidin/biotin, maltose binding protein/maltose, polyhistidine peptide/metal ion such as nickel and cobalt, glutathione-S-transferase/glutathione, calmodulin/calmodulin-binding peptide, ATP binding protein/ATP, receptor protein/ligand and the like.

In addition, as proteins are negatively charged at a pH that is higher than the isoelectric point and positively charged at a pH that is lower than the isoelectric point, a protein can be trapped by using the electric coupling (electrostatic interaction) between the protein and a first protein trapper by using a positive charge material or a negative charge material as the first protein trapper. For instance, a substance having a positively charged group (for instance, an amino group, a guanidyl group and an imidazole group) or a substance having a negatively charged group (for instance, a carboxyl group, a sulfonyl group and a phosphate group) can be used as a positive charge material and a negative charge material. In addition, an electric conductor connected to an electrical circuit can also be used as a positive charge material and a negative charge material.

In addition, a protein can be trapped by using the hydrophobic interaction between a protein and a first protein trapper, by using a substance having a hydrophobic group such as an alkyl group or a derivative group thereof, a phenyl group or a derivative group thereof, and the like, as the first protein trapper.

In each library unit, the first protein trapper is set contiguously to a nucleic acid. The first protein trapper is set so as to be contiguous enough to a nucleic acid contained in the same library unit, rather than a nucleic acid contained in a different species of library unit. In this way, the distance between a nucleic acid and a protein trapper contained in different species of library units becoming considerably larger than the distance between a nucleic acid and a protein trapper contained in the same library unit, a protein expressed by a nucleic acid that is contained in each library unit is trapped preferentially by a protein trapper contained in the same library unit, allowing cross-contamination of a protein expressed in a different species of library unit to be effectively prevented. It is preferred that the first protein trapper is set contiguously to a nucleic acid contained in the same library unit as much as possible, the first protein trapper being set, for instance, at one end of a nucleic acid whose other end is immobilized on a solid support. In this way, the first protein trapper can be set contiguously to a nucleic acid contained in the same library unit with certainty.

The first protein trapper may be immobilized directly on the surface of a solid support, or can be immobilized on a nucleic acid that can express not less than one species of protein in an in vitro transcription/translation system or an in vitro translation system (for instance, among the ends of the nucleic acid, at the end that is on the side opposite to the end immobilized on the solid support). In addition, it may be immobilized on other element that has been immobilized on the surface of the solid support (for instance, a carbohydrate chain or a nucleic acid that can not express a protein in an *in vitro* transcription/translation system (a nucleic acid for immobilizing a first protein trapper onto a solid support)). The quantity of protein expressed and the quantity of protein trapper can be adjusted simply by immobilizing a first protein trapper on other element that has been immobilized on the surface of the solid support.

Immobilization of the first protein trapper can be carried out by a variety of coupling schemes. Specific examples of coupling schemes include specific interaction between streptavidin or avidin and biotin, hydrophobic interaction, magnetic interaction, polar interaction, formation of a covalent bond (for instance, an amide bond, a disulphide bond, a thioether bond and the like), crosslinking by a crosslinking agent and the like. Well known techniques can be used to carry out a suitable chemical modification to the surface of the solid support, the first protein trapper and the like, so that immobilization by these coupling schemes becomes possible. In addition to the specific interaction between streptavidin or avidin and biotin, specific interactions such as maltose binding protein/maltose, polyhistidine peptide/metal ions such as nickel and cobalt, glutathione-S-transferase/glutathione, calmodulin/calmodulin binding peptide, ATP binding protein/ATP, nucleic acid/complementary nucleic acid, receptor protein/ligand, enzyme/substrate, antibody/antigen and IgG/protein A can be used.

In any one or more library units, it is preferred that the first protein trapper be set so as to surround a nucleic acid. As a protein expressed by the nucleic acid contained in the library unit is trapped by a protein trapper (first protein trapper) contained in the same library unit with certainty in a library unit in which a first protein trapper has been set in this way, cross-contamination of a protein expressed in a different species of library unit can be effectively prevented. It is preferred that the number of library unit in which a first protein trapper has been set in this way be as large as possible, the most preferred being that the first protein trapper be set in this way in every library unit.

In case the solid support is a particle, one end of the nucleic acids is immobilized on the surface of the particle and a protein trapper (first protein trapper) is set at the other end of the nucleic acid, if the nucleic acids are immobilized at high density on the surface of the particle, a barrier of protein trappers (first protein trappers) is formed on the outside of the particle contiguously to the nucleic acids, the nucleic acids on the particle being in a state in which they are surrounded by protein trappers (first protein trappers). Therefore, cross-contamination of a protein expressed in a different species of library unit can be effectively prevented by using such a particle as a library unit. Herein, "high density" is a density to the extent that the proteins expressed from the nucleic acids that have been immobilized on a particle do not pass through (do almost not pass through) the barrier of protein trappers formed on the outside of the particle (that is to say a density to the extent that the entirety or almost the entirety of the proteins expressed from the nucleic acids that have been immobilized on the particle are trapped by the protein trappers) , which can be suitably adjusted according to the size or the like of the protein expressed from the nucleic acid that has been immobilized on the particle, and a suitable effect can be obtained by immobilizing, for instance, on the order of 10³ to 10⁶ molecules per 1µm² of the surface of the particle, preferably 10⁵ molecules per 1µm² of the surface of the particle, of nucleic acids.

A protein library comprising plural species of proteins is obtained by subjecting at once not less than two species of library units possessed by the nucleic acid library of the present invention to an *in vitro* transcription/translation system or an *in vitro* translation system to express the nucleic acids contained in each library unit in an *in vitro* transcription/translation system or an *in vitro* translation system. In so doing, the library units to be expressed may be all the library units possessed by the nucleic acid library of the present invention or a portion of the library units possessed by the nucleic acid library of the present invention, as long as there are not less than two species. In addition, the nucleic acids contained in a library unit to be subjected to an *in vitro* transcription/translation system are in general DNA or RNA, and the nucleic acids contained in a library unit to be subjected to an *in vitro* translation system are in general RNA.

When the nucleic acids contained in not less than two species of library units are expressed at once, as the protein trapper contained in each library unit traps preferentially a protein expressed from a nucleic acid contained in the same library unit rather than a protein expressed from a nucleic acid contained in a different species of library unit, cross-contamination of a protein expressed in a different species of library unit is prevented.

When expressing at once the nucleic acids contained in not less than two species of library units, if the mRNA generated by the transcription of the DNA contained in the library unit translocates into the solution of the *in vitro* transcription/translation system, there is a possibility that a protein generated by the translation of the mRNA is trapped by a protein trapper contained in a different species of library unit, giving rise to a cross-contamination of a protein expressed in the different species of library unit. Thus, it is preferred that, in the library unit containing DNA as nucleic acid, an mRNA trapper that can trap an mRNA generated by the transcription of the DNA be set contiguously to the DNA. Note that in case the nucleic acid contained in the library unit is RNA, there is no possibility of cross-contamination of protein caused by an mRNA generated by the transcription of a DNA.

Any mRNA trapper may be suitable as long as it can trap the mRNA. For instance, a nucleic acid (DNA or RNA) that can hybridize with the mRNA can be used as the mRNA trapper. In so doing, the mRNA trapper may hybridize specifically with the mRNA generated by the transcription of the nucleic acid contained in each library unit; however, in such case, a different mRNA trapper becomes necessary for each species of library unit, construction of the nucleic acid library of the present invention becomes difficult. Therefore, generally, an mRNA trapper capable of nonspecifically trapping any mRNA is used. For instance, in case the mRNA generated by the transcription of the DNA contained in the library unit comprises a polyA sequence, an oligonucleotide comprising a polyT sequence can be used as the mRNA trapper. By having a polyT sequence introduced in the DNA contained in the library unit, a polyA sequence can be introduced into the mRNA generated by the transcription of the DNA. The mRNA trap may be directly immobilized on the surface of the solid support, or may be immobilized on the DNA contained in the library unit (for instance, among the ends of the nucleic acid, at the end that is on the side opposite to the end immobilized on the solid support). In addition, it may be immobilized on other member that has been immobilized on the surface of the solid support.

When expressing at once the nucleic acids contained in not less than two species of library units, if a protein expressed from a library unit is not trapped by a protein trapper (first protein trapper) contained in the same library unit and is dispersed into the solution of the *in vitro* transcription/translation system or the *in vitro* translation system, there is a possibility that the protein is trapped by a protein trapper (first protein trapper) contained in a different species of library unit, giving rise to cross-contamination of the protein expressed in the different species of library unit. Thus, it is preferred that a second protein trapper that is separated from the first protein trapper be present, mixed in the solution of the *in vitro* transcription/translation system or the *in vitro* translation system. The species of the second protein trapper may be the same species as or a different species from the species of the primary protein. In the nucleic acid library of the present invention, in case each solid support onto which a library unit has been set (for instance a particle) is present in a state in which it is dispersed in a liquid, the second protein trapper can be present, mixed in the liquid. The second protein trapper may exist alone; however it is preferred that it be immobilized on a solid support (for instance a particle), so as to be easily separated by post-processing.

Any second protein trapper is suitable as long as it is capable of trapping a protein expressed from each library unit. Generally, a protein trapper capable of nonspecifically trapping any protein is used as the second protein trapper. As is the case with the first protein trapper, a protein trapper that can nonspecifically trap any protein by chemical coupling, electric coupling or physical coupling can be used as a second protein trapper. In case a protein expressed from a library unit is a fusion protein between a target protein and a tag protein that can bind to the first protein trapper, it is preferred that the tag protein can also bind to the second protein trapper. In this way, a fusion protein that has been dispersed into the solution of an *in vitro* transcription/translation system or an *in vitro* translation system can be trapped with certainty by the second protein trapper.

When expressing at once the nucleic acids contained in not less than two species of library units, if excessive mRNA is expressed from the DNA contained in the library unit and this is translocated into the solution of an *in vitro* transcription/translation system, there is a possibility that a protein generated by the translation of the mRNA is trapped by a protein trapper contained in a different species of library unit, giving rise to cross-contamination of a protein expressed in a different species of library unit. Thus, in order to prevent excessive expression of mRNA, it is preferred to express at once the aforementioned nucleic acids while inhibiting the transcription reaction in the *in vitro* transcription/translation system. However, as proteins can no longer be expressed from each library unit if the transcription reaction is completely inhibited, the transcription reaction must not be completely inhibited. Note that in case the nucleic acid contained in the library unit is RNA, there is no possibility of cross-contamination of protein caused by an excessive expression of mRNA.

Inhibition of the transcription reaction in the in vitro transcription/translation system can be carried out by a quantitative inhibition or a qualitative inhibition of the RNA polymerase contained in the *in vitro* transcription/translation system.

A "quantitative inhibition of the RNA polymerase" means to decrease the quantity of RNA polymerases that can participated in the transcription reaction among the RNA polymerases contained in the *in vitro* transcription/translation system, and the quantitative inhibition of the RNA polymerase can be carried out, for instance, by having an RNA polymerase binder present, mixed in the in vitro transcription/translation system. Any RNA polymerase binder is suitable as long as it can bind to the RNA polymerase (reversible binding or irreversible binding), for instance, a nucleic acid having an RNA polymerase binding site (for instance a promoter region), or a solid support (for instance a particle) on the surface of which the nucleic acid has been immobilized, can be used as the RNA polymerase binder.

A "qualitative inhibition of the RNA polymerase" means to decrease the reactivity of the individual RNA polymerase contained in the in vitro transcription/translation system, and the qualitative inhibition of the RNA polymerase can be carried out, for instance, by adjusting the temperature of the *in vitro* transcription/translation system. The temperature of *in vitro* transcription/translation system can be increased or decreased as long as the transcription reaction can be inhibited. In case the transcription reaction is to be inhibited by increasing the temperature of the *in vitro* transcription/translation system to higher than the optimal temperature of the transcription reaction, the temperature of the *in vitro* transcription/translation system is adjusted in general to between 34 and 42°C and preferably to between 36 and 38°C. Note that there is a possibility that not only the transcription reaction but also the translation reaction will be inhibited by adjusting the temperature of the *in vitro* transcription/translation system; in such a case, the inhibition of the translation reaction can be prevented by pre-including in the *in vitro* transcription/translation system larger amounts of factors that are involved in the translation reaction.

In the nucleic acid library of the present invention, in case the particles onto which each library unit has been set are in a state in which they are dispersed in a liquid, a protein trapper contained in each library unit becomes capable of trapping preferentially a protein expressed from a nucleic acid contained in the same library unit rather than a protein expressed from a nucleic acid contained in a different species of library unit, by increasing the distance between the particles. Therefore, it is preferred to adjust the concentration of the particles to a low concentration, in order to increase the distance between the particles onto which each library unit has been set. A satisfactory effect can be obtained by adjusting the particle concentration to 10⁴ to 10⁵ per 50µL.

In addition, it is preferred that particles onto which no nucleic acid that can express a protein in an *in vitro* transcription/translation system or an *in vitro* translation system has been immobilized is present, mixed in the liquid in which the particles are dispersed, in order to increase the distance between the particles onto which each library unit has been set. By having such particles mixed in, the problem of decrease in handling ability of the particles that arises when the particle concentration in the liquid is at low concentration can also be eliminated, while allowing the distance between the particles onto which each library unit has been set to be increased. Note that a nucleic acid that can not express a protein in an *in vitro* transcription/translation system or an *in vitro* translation system may be immobilized on the surface of the particles to be mixed in. By having such a nucleic acid immobilized on the surface of the particles to be mixed in, unexpected influence onto the reaction system due to the mixing of the particles can be prevented, such as a protein necessary for transcription/translation contained in an *in vitro* transcription/translation system or *in vitro* translation system or a protein expressed from each library unit being nonspecifically adsorbed by the particles.

In case a particle that is different from the particles onto which each library unit has been set has been mixed in the liquid, both particles can be fractionated for instance by flow cytometry, by differentiating the diameters of the particles. In addition, by using a particle having magnetism as the particle onto which each library unit has been set, and using a particle that does not have magnetism as the different particle to be mixed in, both particles can be fractionated based on the difference in magnetization when a magnet is approached.

In the nucleic acid library of the present invention, since cross-contamination of a protein expressed in a different species of library unit is prevented even if nucleic acids contained in not less than two species of library units are expressed at once, the protein expressed from a nucleic acid that is contained in each library unit is displayed on the solid support in a state in which it is trapped by a protein trapper (first protein trapper) contained in the same library unit. That is to say, in the protein library of the present invention, each species of protein is displayed on the solid support in a state in which it is correlated with the nucleic acid coding therefor.

In the nucleic acid library of the present invention, in case all the library units are set on the surface of a single solid support, each species of protein is displayed on the single solid support when the solid support is immersed in a solution of an *in vitro* transcription/translation system or an *in vitro* translation system. In addition, in case different species of library units are respectively set on the surface of separate solid supports and dispersed in a liquid, different species of proteins are respectively displayed on the separate solid supports, by adding elements of an *in vitro* transcription/translation system or an *in vitro* translation system into the liquid. Analyses of plural species of proteins can be performed effectively in parallel by using the protein library of the present invention created in this way. In addition, since the nucleic acid library of the present invention is immediately converted into a protein library when subjected to an *in vitro* transcription/translation system or an *in vitro* translation system, the supply of protein and the protein assay system are integrated, and analyses of plural species of proteins can be started immediately after supplying the plural species of proteins.

Protein analyses using the protein library of the present invention can be carried out, for instance, as follows.

In the protein library of the present invention, in case each species of proteins are displayed on a single solid support, the solid supports are isolated from the solution of an *in vitro* transcription/translation system or an *in vitro* translation system and washed to remove proteins and other contaminants from the *in vitro* transcription/translation system or the *in vitro* translation system. Thereafter, each species of protein displayed on the solid support and a labeled target substance (for instance, a protein, a nucleic acid, a carbohydrate, a lipid and the like) are reacted. After the reaction, the solid supports are washed to remove unreacted target substances, and whether each species of protein displayed on the solid support and the target substance reacted can be detected by detecting the label of the target substance. The species of the protein with which the target substance reacted can be identified by the location or the like where the protein is displayed. In addition, the amino acid sequence of the protein can be identified by analyzing the base sequence of the nucleic acid that is correlated with the protein.

In the protein library of the present invention, in case different species of proteins are respectively displayed on separate magnetic particles and each magnetic particle is dispersed in the solution of an *in vitro* transcription/translation system or an *in vitro* translation system, after separating each magnetic particle from the solution of the *in vitro* transcription/translation system or the *in vitro* translation system by applying a magnetic force, each magnetic particle is transferred into a washing solution and washed to remove proteins and other contaminants from the *in vitro* transcription/translation system or the *in vitro* translation system. Thereafter, each magnetic particle is added to a solution containing the labeled target substance (for instance, a protein, a nucleic acid, a carbohydrate, a lipid and the like) and agitated thoroughly, to react each species of protein displayed on each magnetic particle with the labeled target substance. After the reaction, a magnetic force is applied to separate each magnetic particle from the solution, each magnetic particle is transferred into a washing solution and washed, to remove unreacted target substances. Thereafter, whether each species of protein displayed on the magnetic particle and the target substance reacted can be detected by detecting the label of the target substance. The species of the protein with which the target substance reacted can be identified by the label or the like of the magnetic particle on which the protein is set. In addition, the amino acid sequence of the protein can be identified by analyzing the base sequence of the nucleic acid that is correlated with the protein.

In the protein library of the present invention, in case different species of proteins are respectively displayed on separate particles, and each particle is dispersed in a solution of an *in vitro* transcription/translation system or an *in vitro* translation system, by reacting each particle and an antibody labeled with a specific fluorescent dye corresponding to a protein species, then fractionating the particles labeled with the fluorescent dye by flow cytometry, and analyzing the base sequence of the nucleic acid that has been immobilized on the fractionated particles, the antigen to which the antibody bound can be rapidly specified.

In the protein library of the present invention, in case different species of proteins are respectively displayed on separate particles, the gene coding for the protein can be concentrated using the properties of the protein presented on each particle.

Even if a protein contained in the protein library of the present invention has been denatured or inactivated in the course of protein analysis, the protein can be regenerated by re-expressing the nucleic acid contained in the library unit on which the protein is displayed. In addition, after the end of the protein analyses, the library can be conserved in the state of a nucleic acid library until the next time of use, and used when necessary by expressing the nucleic acids contained in each library unit to regenerate the protein library.

In the following, the present invention will be explained in further detail based on examples.

### [Example 1]

(1) Preparation of DNA- immobilized beads for protein synthesis

The 5' side of the avidin gene (Thompson & Weber, Gene, 136, 243-246, 1993; refer to SEQ ID NO: 1) was labeled with one of two different species of tags. Histidine (hereinafter called "His") and the HA peptide derived from the influenza hemagglutinin protein (hereinafter called "HA") were used as the two species of tags.

Following the protocols of Rapid Translation System RTS 100 and the E. coli HY Kit (manufactured by Roche), the T7 promoter and a ribosomal binding region (ribosome binding site (RBS)) on the 5' side of the His-labeled avidin gene and the HA-labeled avidin gene and a T7 terminator on the 3' side were introduced by PCR. After cloning the PCR product into a pGEM T easy vector (manufactured by Promega) and confirming the base sequence, a biotinylation primer, which hybridizes specifically upstream from the T7 promoter, and a primer for introducing an SfiI site, which hybridizes specifically downstream from the T7 terminator, were designed. These primers are shown indicated by 17bio-f and sfi-trm in FIG. 1 (A), or 11bio-r and sfi-prm in FIG. 1 (B). Note that the structure upstream from the start codon of the avidin gene portion in the His-labeled avidin gene is shown in FIG. 1 (C) , the structure upstream from the start codon of the avidin gene portion in the HA-labeled avidin gene is shown in FIG. 1 (D) and the structure downstream from the termination codon of the avidin gene portion in the His-labeled avidin gene and the HA-labeled avidin gene is shown in FIG. 1 (E) . The underlined portions in FIG. 1 (C) indicate, in order from the 5' end side, the T7 promoter, g10 (an RBS enhancer) , RBS, ATG and His. The underlined portions in FIG. 1 (D) indicate, in order from the 5' end side, the T7 promoter, g10 (an RBS enhancer), RBS, ATG and HA. The underlined portion in FIG. 1 (E) indicates the T7 terminator. The bold-letter portions in FIG. 1 (A) to (E) indicate the sequences that each primer specifically binds to. Note that the expression vector pIVEX2.4a recommended by RTS100 was used as a reference for the sequences of the T7 promoter and the like.

PCR was carried out using the above-mentioned primers with the His-labeled avidin gene and the HA-labeled avidin gene as templates, to prepare a DNA fragment possessing biotin (17bio-f) on the 5' side and an SfiI site (sfi-trm) on the 3' side. Note that a DNA fragment possessing biotin (11bio-r) on the 3' side and an SfiI site (sfi-prm) on the 5' side was also prepared, with successful protein expression.

After purifying 50µL (approximately 1µM) of each DNA fragment with MicroSpin S-400 HR Columns (manufactured by Amersham Pharmacia Biotech) and digesting with the restriction enzyme SfiI (50°C, 1 hours 30 minutes), each DNA fragment (approximately 50pmol) was recovered by the biotin-avidin reaction (15 minutes in lxBW buffer solution at room temperature), using 0.2mg of Dynabeads M-280 Streptavidin (manufactured by Dynalbiotech) that had been washed with 1×BW buffer (5mM Tris-HCl pH7.5, 0.5mM EDTA, 1M NaCl) according to the protocol. In this way, beads on the surface of which each DNA fragment has been immobilized were obtained via the biotin-avidin reaction.

The various beads were washed with 1×BW buffer, and then suspended in 1×T4 DNA ligation buffer (manufactured by TAKARA), in order to ligate with an adapter.

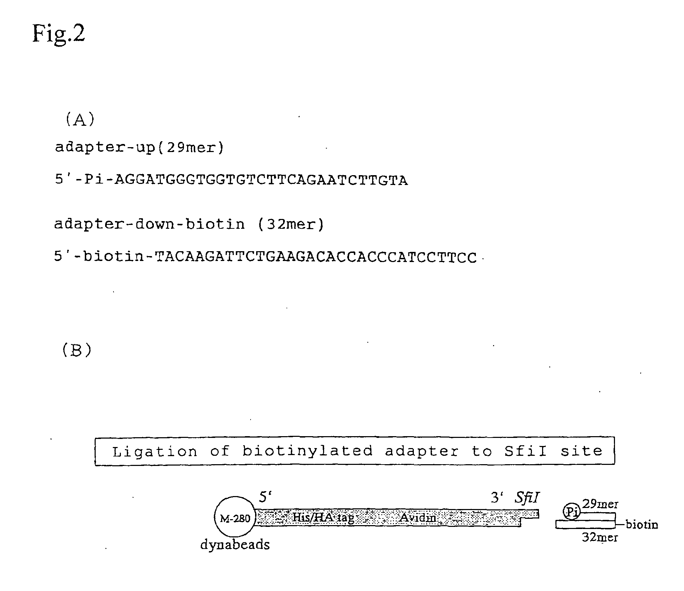

Meanwhile, a biotinylated adapter for ligation to the SfiI site was prepared by mixing equal quantities of a synthetic oligo DNA phosphorylated on the 5' side and a synthetic oligo DNA having an adequate sequence and biotinylated on the 5' side, and annealing the two (cooling from 85°C to 4°C over 1 hour 30 minutes). The base sequence of the adapter is shown in FIG. 2 (A). In addition, the way the biotinylated adapter is ligated to the SfiI site is shown schematically in FIG. 2 (B).

Using TaKaRa T4 DNA ligase (525 units), 0. 2mg of beads to which DNA fragments (approximately 50pmol) cut with SfiI have been bound and the biotinylated adapter (240pmol) prepared by annealing the synthetic oligo DNAs were ligated via a reaction of one hour at room temperature with slight mixing.

A template DNA was prepared via the above operations whose 5' end side is immobilized on the bead (0.2 mg) and possessing biotin on the 3' end side. That is to say, two species of beads were prepared, i.e., a bead on the surface of which a DNA fragment coding for His-avidin has been immobilized (hereinafter called "His-avidin bead"), and a bead on the surface of which a DNA fragment coding for HA-avidin has been immobilized (hereinafter called "HA-avidin bead"). The sequences of the DNA fragments retained by these two species of beads are shown in SEQ ID NO: 2 for His-avidin and SEQ ID NO: 3 for HA-avidin. After washing these two species of beads respectively in 1×BW buffer, they were suspended in 40µL of TE (10mM Tris-HCl pH7.5, 1mM EDTA pH8.0).

Beads obtained by immobilizing a double stranded DNA onto a streptavidin immobilization magnetic beads (particle diameter: 0.7µm) (contained in TOYOBO: MagExtractor-Sequencing Clean up), which has a different size than Dynabeads M-280 Streptavidin (particle diameter: 2.8µm), were prepared (hereinafter "sav beads") as beads to coexist during the synthesis of proteins from the His-avidinbeads or the HA-avidin beads. The double stranded DNA was prepared by mixing sav-comp and sav-Biotin biotinylated on the 3' side shown in FIG. 1 (F), and then annealing by cooling from 85°C to 4°C over 1 hour 30 minutes. The prepared double stranded DNA (10nmol) was immobilized onto the surface of the beads (2mg) via a biotin-avidin reaction in 1×BW Buffer, washed with 1×BW Buffer and then suspended in TE 400µL. Note that the double stranded DNA immobilized on the savbeads contains absolutelyno sequence that is necessary for transcription and translation. There is a possibility that, by coexistence of sav beads, an unexpected influence is exerted on the reaction, such as a protein necessary for transcription/translation contained in the *in vitro* transcription/translation system, or a protein synthesized from a His-avidin bead or a HA-avidin bead is nonspecifically adsorbed onto the sav beads; however, such a possibility can be decreased by also having a double stranded DNA immobilized on the sav beads in the same way as for the His-avidin beads or the HA-avidin beads.

(2) Protein synthesis (*in vitro* transcription/translation)

Protein synthesis was carried out by an in vitro transcription/translation system, using the DNA-immobilized beads for protein synthesis prepared as described above. A protein (a fusion protein between His and avidin, or a fusion protein between HA and avidin) was synthesized in 10µL total volume of, respectively, a solution containing only His-avidin beads, a solution containing only HA-avidin beads, and a solution containing mixed beads of His-avidin beads and HA-avidin beads, by a reaction of 2 hours or 4 hours at 30°C with the DNAs on the beads as templates, following the RTS100 protocol. Note that 45µg of sav beads was added in all the reaction solutions.

(3) Detection and identification by fluorescently labeled antibody

After washing various beads with 1×BW buffer, blocking was performed with a PBS-T (137mM NaCl, 2.7mM KCl, 4.3mM Na₂HPO₄·7H₂O, 1.4mM KH₂PO₄, 0.05% Tween 20) + 5% skim milk solution for 1 hour at room temperature with slight mixing. Then, after washing with PBS-T, primary and secondary antibody reactions were carried out for hour each at room temperature with slight mixing. For primary antibodies, 0.5µg each of anti-His antibody derived from mouse (manufactured by Amersham Pharmacia Biotech) and anti-HA antibody derived from rabbit (Manufactured by Cosmobio) were diluted in 100µL of PBS-T and used. After washing with PBS-T, 0.5µg of goat anti-mouse IgG antibody derived from goat labeled with Alexafluor488 (manufactured by Funakoshi) was diluted in 100µL PBS-T and used as a secondary antibody for fluorescently labeling the anti-His antibody. After washing with PBS-T, measurements were made with a flow cytometer (FACS Calibur (manufactured by Becton Dickinson)), at an excitation wavelength of 488nm, and measurement wavelengths of 530/30nm.

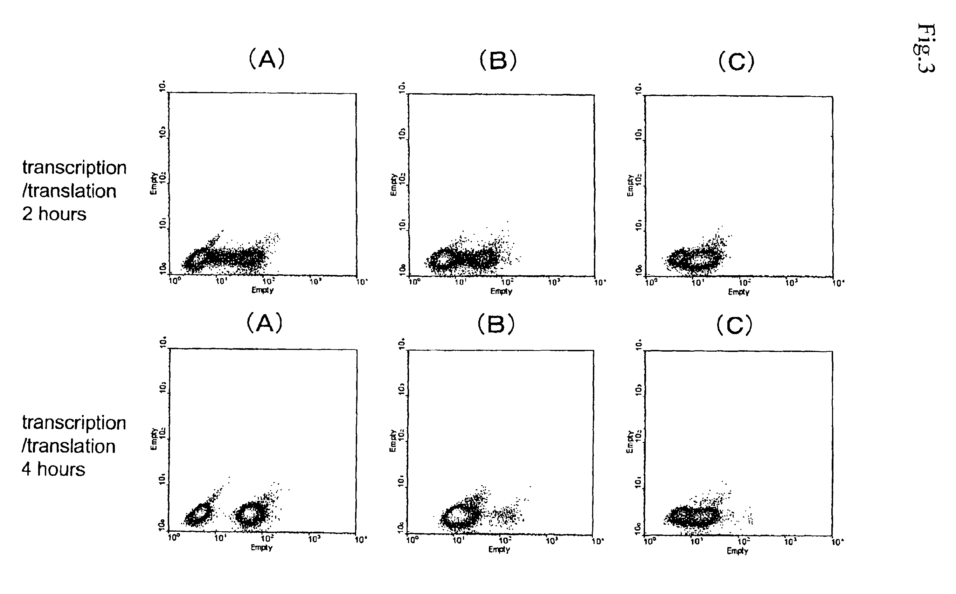

The results of the measurements are shown in FIG. 3. In FIG. 3, A shows the results obtained when a solution containing only His-avidin beads and a solution containing only HA-avidin beads as the DNA- immobilized beads for protein synthesis were used, the respective proteins were expressed, then mixed and the above-mentioned measurements were carried out, B shows the results obtained when a solution containing mixed beads of His-avidin beads and HA-avidin beads as the DNA- immobilized beads for protein synthesis was used to express proteins, and the above-mentioned measurements were carried out, and C shows the results obtained when a solution containing mixed beads of His-avidin beads and HA-avidin beads as the DNA-immobilized beads for protein synthesis was used to express proteins and biotinylated agarose gel was mixed in the solution to trap the proteins present in the solution

As shown in FIG. 3 A and B, when the transcription/translation time was 2 hours, two species of beads were obtained, with different fluorescence intensities. One of these two species of beads is thought to be a bead in which a fusion protein between His and avidin has been trapped, the other is thought to be a bead in which a fusion protein between HA and avidin has been trapped. Considering the results from the following Example 3, since these two species of beads are thought to be His-avidin beads and HA-avidin beads, the fusion protein between His and avidin is thought to have been trapped by the His-avidin beads, and the fusion protein between HA and avidin are thought to have been trapped by the HA-avidin beads.

From the foregoing, it is thought that when beads are present in a state in which they are dispersed in the liquid (that is to say, when they are separated from one another), a protein trapper immobilized on a bead (biotin in the present Example) can trap preferentially a protein expressed by a DNA fragment that has been immobilized on the same bead (a fusion protein between His and avidin or a fusion protein between HA and avidin in the present Example), rather than a protein expressed by a DNA fragment that has been immobilized on a different bead (a fusion protein between His and avidin or a fusion protein between HA and avidin in the present Example), thereby preventing cross-contamination of proteins between beads on which different species of DNA fragments have been immobilized.

However, a clear separation of beads was not observed when the transcription/translation time was 4 hours. It is thought that the reason is the release into the solution and translation of an excess of mRNA generated by the transcription, due to the elongation of the transcription/translation time. Therefore, it is thought that cross-contamination of protein can be prevented between beads on which different species of DNA fragments have been immobilized, by mixing a protein trapper in a solution to carry out transcription/translation. Actually, in case biotinylated agarose gel was present, mixed in the solution (FIG. 3C) a clearer separation of the beads was observed than without mixing (FIG. 3B).

From the foregoing, it is thought that cross-contamination of protein can be prevented between beads on which different species of DNA fragments have been immobilized, by having a protein trapper (biotinylated agarose gel in the present Example) that can trap a protein expressed from various beads (a fusion protein between His and avidin or a fusion protein between HA and avidin in the present Example) mixed in a solution where transcription/translation is to be carried out.

### [Example 2]

(1) Preparation of DNA-immobilized beads for protein synthesis

DNA-immobilized beads for protein synthesis were prepared in the same way as in Example 1.

(2) Protein synthesis *(in vitro* transcription/translation)

Protein synthesis was carried out by an *in vitro* transcription/translation system, using the DNA-immobilized beads for protein synthesis prepared as described above. A protein (a fusion protein between His and avidin, or a fusion protein between HA and avidin) was synthesized in 10µL total volume of, respectively, a solution containing only His-avidin beads, a solution containing only HA-avidin beads, and a solution containing mixed beads of His-avidin beads and HA-avidin beads, by a reaction of one hour or 1 hour and 15 minutes at 30°C, or 1 hour and 45 minutes at 37°C with the DNAs on the beads as templates, following the RTS100 protocol. Note that 45µg of sav beads was added in all the reaction solutions.

In addition, 50 pmol of a DNA fragment having a T7 promoter sequence (17mer and 60mer) (hereinafter called "inhibitor DNA") was added in order to inhibit the transcription reaction by the T7 RNA polymerase. The base sequences of the 17mer and the 60mer inhibitor DNAs are shown respectively in SEQ ID NO: 4 and 5.

(3) Detection and identification by fluorescently labeled antibody

After washing various beads with 1×BW buffer, blocking was performed with a PBS-T (137mM NaCl, 2.7mM KCl, 4.3mM Na₂HPO₄·7H₂O, 1.4mM KH₂PO₄, 0.05% Tween 20) + 5% skim milk solution for 1 hour at room temperature with slight mixing. Then, after washing with PBS-T, primary and secondary antibody reactions were carried out for hour each at room temperature with slight mixing. Monoclonal anti-HA antibody derived from mouse (Manufactured by Molecular Probe) labeled with Alexafluor488 and anti-His antibody derived from rabbit (manufactured by Cosmobio) were used as primary antibodies, anti-rabbit IgG antibody derived from goat (manufactured by Funakoshi) labeled with phycoerythrin (PE) was used as secondary antibody. For each antibody, 0.5µg each was diluted in 100µL of PBS-T in case of primary antibodies, 2µg was diluted in 100µL PBS-T in case of secondary antibody, and used. After binding of each antibody, the beads were washed with PBS-T. Measurements were made with a flow cytometer (FACS Calibur (manufactured byBecton Dickinson) ) , at an excitation wavelength of 488nm, and measurement wavelengths of 530/30nm and 585/45nm.

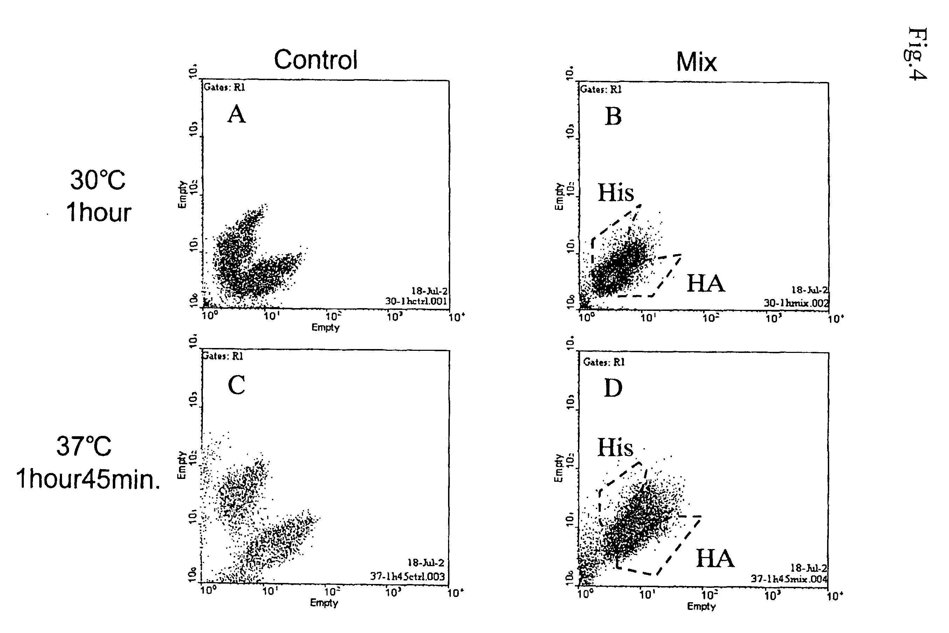

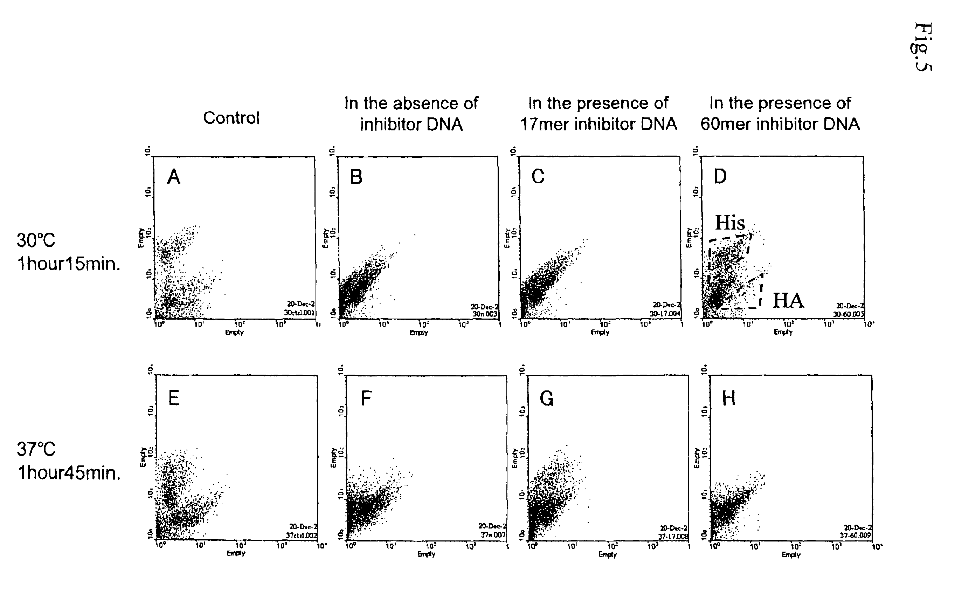

The results of the measurements are shown in FIG. 4 and 5.

In FIG. 4, "control" (A and C) show the results obtained when a solution containing only His-avidin beads and a solution containing only HA-avidin beads were used, the respective proteins were expressed, then mixed and the above-mentioned measurements were carried out, "Mix" (B and D) shows the results obtained when a solution containing mixed beads of His-avidin beads and HA-avidin beads was used to express proteins, and the above-mentioned measurements were carried out. Note that the transcription/translation conditions for A and B were 1 hour at 30°C and the transcription/translation conditions for C and D were 1 hour 45 minutes at 37°C.

In FIG. 5, "control" (A and E) shows the results obtained when a solution containing only His-avidin beads and a solution containing only HA-avidin beads, the respective proteins were expressed, then mixed and the above-mentioned measurements were carried out, "in the absence of inhibitor DNA" (B and F), "in the presence of 17mer inhibitor DNA" (C and G) and "in the presence of 60mer inhibitor DNA" (D and H) show the results obtained when a solution containing mixed beads of His-avidin beads and HA-avidin beads (no addition of inhibitor DNA, addition of 17mer inhibitor DNA and addition of 60mer inhibitor DNA) was used to express proteins, and the above-mentioned measurements were carried out. Note that the transcription/translation conditions for A, B, C and D were 1 hour 15 minutes at 30°C, andthe transcription/translation time for E, F, G and H was 1 hour 45 minutes at 37°C.

As shown in FIG. 4 B and D, although the quantity of translation product appears to be slightly decreased at 37°C than at 30°C, a clear separation of beads was observed at 37° than at 30°C. In addition, as shown in FIG. 5B, in the absence of inhibitor DNA, no clear separation of beads was observed; however, a clear separation of beads was observed at 37°C in the presence of 17mer inhibitor DNA as shown in FIG. 5 C and G, and at 30°C in the presence 60mer inhibitor DNA as shown in FIG. 5D and H.

From the fact that a clear separation of beads indicates that two species of beads have been obtained, i.e., beads where a fusion protein between His and avidin has been trapped and beads where a fusion protein between HA and avidin has been trapped, as well as, the fact that the presence of an inhibitor DNA and the increase in the reaction temperature are both factors that inhibit the transcription reaction, it is thought that cross-contamination of protein between the beads can be prevented by inhibiting the transcription reaction in the *in vitro* transcription/translation system.

Note that, no clear separation of beads was observed at 37°C in the presence 60mer inhibitor DNA as shown in FIG. 5H; however, from the fact that the addition of inhibitor DNA and a reaction temperature of 37°C both provoke a decrease in the quantity of protein translated, the reason may be that in this condition, almost no protein is generated and the beads have not moved from the origin.

### [Example 3]

In order to confirm that the two species of beads separated by flow cytometry in Example 2 (beads in which the fusion protein between His and avidin has been trapped and beads in which fusion protein between HA and avidin has been trapped) are His-avidin beads and HA-avidin beads, the beads in the regions delimited by the dotted lines in FIG. 4B and D as well as in FIG. 5D were fractionated. In FIG. 4B and D as well as in FIG. 5D, the regions in which His-avidin beads were expected to be contained were marked by "His", and the regions in which HA-avidin beads were expected to be contained were marked by "HA".

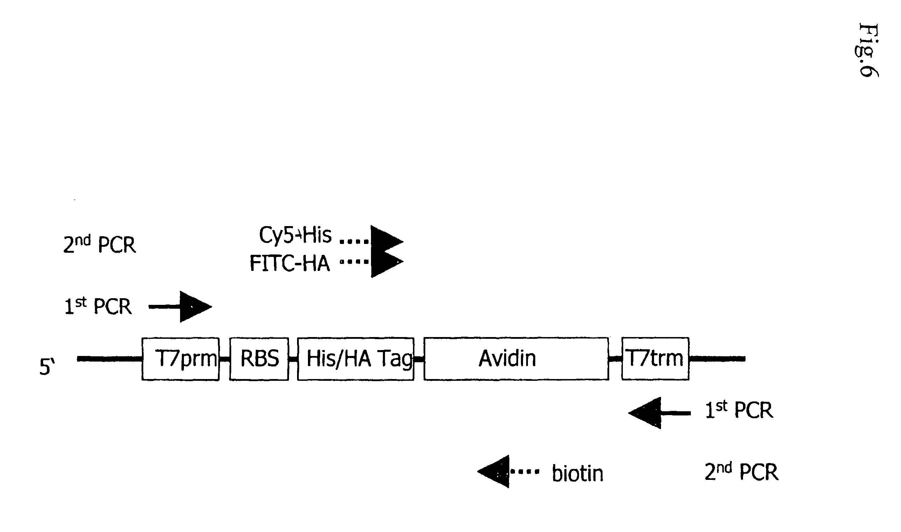

Determination of the species of DNAs immobilized on the surface of the beads that have been fractionated as described above was carried out using PCR. The locations where primers have been designed are shown in FIG. 6.

First, amplification was carried out, using primers for the 1st PCR with the fractionated beads as templates. The base sequences of the upstream and downstream primers used in the 1st PCR are shown respectively in SEQ ID NO: 6 and 7. Next, amplification was carried out using primers for the 2nd PCR, with the products of the 1st PCR as templates. In the 2nd PCR, a mixture of two species of primers labeled with Cy5 or FITC (Cy5-His and FITC-HA) were used as primers on the upstream side, and a biotinylated primer was commonly used as the primer on the downstream side. The base sequence of the primers Cy5-His and FITC-HA are shown respectively in SEQ ID NO: 8 and 9, and the base sequence of the biotinylated primer is shown in SEQ ID NO: 10.

With the above PCR, DNA fragments whose ends are labeled with FITC and biotin are amplified in case the DNA fragments on the beads have a base sequence coding for an HA tag, and DNA fragments whose ends are labeled with Cy5 and biotin are amplified in case the DNA fragments on the beads have a base sequence coding for a His tag. Therefore, the species of the DNA fragments that were used as templates in the 1st PCR can be determined by trapping the DNA fragments that were amplified by the 2nd PCR with avidin magnetic beads and measuring the fluorescence intensity of the fluorescent dye (FITC or Cy5) trappedonthemagneticbeads.

The results of measurements of the fluorescence intensities are shown in Table 1 and 2.

**[Table 1]**

| | FITC | Cy5 | FITC/Cy5 |
|---|---|---|---|
| His:HA=1:9 | 26.73 | 1.38 | 19.34 |
| His:HA=1:1 | 15.14 | 4.04 | 3.75 |
| His:HA=9:1 | 3.22 | 5.99 | 0.54 |

**[Table 2]**

| | FITC | Cy5 | FITC/Cy5 |
|---|---|---|---|
| FIG. 4B-His | 3.88 | 3.69 | 1.05 |
| FIG. 4B-HA | 14.72 | 1.11 | 13.27 |
| FIG. 4D-His | 5.87 | 4.80 | 1.22 |
| FIG. 4D-HA | 17.87 | 1.57 | 11.37 |
| FIG. 5D-His | 3.79 | 3.14 | 1.21 |
| FIG. 5D-HA | 20.94 | 1.55 | 13.55 |

Table 1 shows the results obtained when Cy5-His and FITC-HA were mixed in proportions of respectively 1:9, 1:1 and 9:1 as a comparative experiment, and Table 2 shows the results obtained when the beads that were fractionated from the regions delimited by the dotted lines in FIG. 4B and D as well as FIG. 5D were used as templates. In Table 2, FIG. 4B-His, FIG. 4D-His and FIG. 5D-His represent the beads that were fractionated from the regions marked "His" in FIG. 4B, FIG. 4D and FIG. 5D, respectively, and FIG. 4B-HA, FIG. 4D-HA and FIG. 5D-HA represent the beads that were fractionated from the regions marked "HA" in FIG. 4B, FIG. 4D and FIG. 5D, respectively.

As shown in Table 2, the FITC/Cy5 ratio for the beads that were expected to be His-avidin beads is small, and the FITC/Cy5 ratio for the beads that were expected to be HA-avidin beads were large. Therefore, it was confirmed that the two species of beads that were separated by flow cytometry in Example 2 were His-avidin beads and HA-avidin beads.

From the above results, it was confirmed that a fusion protein between His and avidin is trapped by His-avidin beads, and a fusion protein between HA and avidin is trapped by HA-avidin beads.

### INDUSTRIAL APPLICABILITY

According to the present invention, a nucleic acid library, a protein library and a method for making a protein library are provided, allowing plural species of proteins to be supplied at once in a state in which they are correlated with the nucleic acids coding therefor, while allowing analyses of plural species of proteins to be performed in parallel immediately after supplying the plural species of proteins, and furthermore allowing a denatured or inactivated protein to be regenerated easily. In addition, according to the present invention, a particle that is useful as a library unit to constitute a nucleic acid library and a protein library is provided.

## Claims

1. A nucleic acid library comprising not less than two species of library units present in a state in which they are separated from one another, wherein
each library unit comprises a nucleic acid that can express not less than one species of protein in an *in vitro* transcription/translation system or an *in vitro* translation system, and a first protein trapper that is set contiguously to the nucleic acid,
each library unit is set on the surface of a solid support, and the nucleic acid contained in each library unit is immobilized on the surface of the solid support where each library unit is set.

2. The nucleic acid library as claimed in Claim 1, wherein all the library units are set on the surface of a single solid support.

3. The nucleic acid library as claimed in Claim 2, wherein the position of each library unit on the surface of the solid support is correlated with the species of each library unit.

4. The nucleic acid library as claimed in Claim 2 or 3, wherein the solid support has a shape that can be wound around an axial member.

5. The nucleic acid library as claimed in Claim 1, wherein different species of library units are respectively set on the surface of separate solid supports.

6. The nucleic acid library as claimed in Claim 5, wherein the solid supports onto which different species of library units are set are mutually distinct and identifiable.

7. The nucleic acid library as claimed in Claim 5 or 6, wherein the solid support is a particle.

8. The nucleic acid library as claimed in Claim 7, wherein the particle possesses magnetism.

9. The nucleic acid library as claimed in Claim 7 or 8, wherein the particle is dispersed in a liquid.

10. The nucleic acid library as claimed in Claim 9, wherein a particle onto which no nucleic acid that can express a protein in an in vitro transcription/translation system or an *in vitro* translation system is immobilized is mixed in the liquid.

11. The nucleic acid library as claimed in Claim 9 or 10, wherein a second protein trapper or an RNA polymerase binder is mixed in the liquid.

12. The nucleic acid library as claimed in any of Claim 1 to 11, wherein the solid support is porous.

13. The nucleic acid library as claimed in Claim 12, wherein the solid support is a fiber or an aggregate thereof.

14. The nucleic acid library as claimed in any of Claim 1 to 13, wherein in any one or more of the library units, one end of the nucleic acid is immobilized onto the solid support and the first protein trapper is set at the other end of the nucleic acid.

15. The nucleic acid library as claimed in any of Claim 1 to 14, wherein the first protein trapper is set so as to surround the nucleic acid in any one or more library units.

16. The nucleic acid library as claimed in any of Claim 1 to 15, wherein the protein that can be expressed by the nucleic acid in any one or more library units is a fusion protein between a target protein and a tag protein that can bind to the first protein trapper.

17. The nucleic acid library as claimed in Claim 16, wherein the tag protein can bind to the second protein trapper.

18. The nucleic acid library as claimed in any of Claim 1 to 17, wherein in the library unit containing a DNA as the nucleic acid, an mRNA trapper is set contiguously to the DNA.

19. A protein library obtainable by subjecting at once not less than two species of library units possessed by the nucleic acid library as claimed in any of Claim 1 to 18 to an in vitro transcription/translation system or an *in vitro* translation system to express at once the nucleic acids contained in the library units.

20. Amethod for making a protein library, comprising
subjecting at once not less than two species of library units possessed by the nucleic acid library as claimed in any of Claim 1 to 18 to an *in vitro* transcription/translation system or an *in vitro* translation system to express at once the nucleic acids contained in the library units.

21. The method for making a protein library as claimed in Claim 20, wherein a second protein trapper is mixed in the in vitro transcription/translation system or the in vitro translation system.

22. The method for making a protein library as claimed in Claim 20 or 21, wherein the nucleic acids are expressed at once while the transcription reaction in the in vitro transcription/translation system is being inhibited.

23. The method formaking a protein library as claimed in Claim 22, wherein an RNA polymerase binder is mixed in the *in vitro* transcription/translation system to inhibit the transcription reaction in the *in vitro* transcription/translation system.

24. The method for making a protein library as claimed in Claim 22 or 23, wherein the transcription reaction in the *in vitro* transcription/translation system is inhibited by adjusting the temperature of the *in vitro* transcription/translation system.

25. A kit for making a protein library, comprising the nucleic acid library as claimed in any of Claim 1 to 18 and a second protein trapper or an RNA polymerase binder.

26. A particle on the surface of which nucleic acids that can express not less than one species of protein in an *in vitro* transcription/translation system or an *in vitro* translation system are immobilized at high density, wherein one end of each nucleic acid is immobilized on the surface of the particle and a protein trapper is set at the other end of each nucleic acid.


SEQUENCE LISTING

<110> Precision System Science Co., Ltd
National Institute of Advanced Industrial Science and Technology

<120> nucleic acid library and protein library

<130> EP 03745446.9

<150> JP 2002-095285
<151> 2002-03-29

<160> 10

<210> 1
<211> 390
<212> DNA
<213> Streptomyces avidinii

<220>
<221> CDS
<222> (1)..(390)

<400> 1
atg gct gaa gct ggt atc acc ggt act tgg tac aac cag ctg ggg tct 48
Met Ala Glu Ala Gly Ile Thr Gly Thr Trp Tyr Asn Gln Leu Gly Ser
1               5                   10                  15

acc ttc atc gtt acc gct ggt gct gac ggt gca ctg acc ggt act tac 96

Thr Phe Ile Val Thr Ala Gly Ala Asp Gly Ala Leu Thr Gly Thr Tyr
            20                  25                  30

gaa agc gct gtt ggt aac gct gaa agc cgt tat gtt ctg acc ggt cgt 144
Glu Ser Ala Val Gly Asn Ala Glu Ser Arg Tyr Val Leu Thr Gly Arg
        35                  40                  45

tac gac tct gct ccg gct acc gac ggt tct ggt act gct ctg ggt tgg 192
Tyr Asp Ser Ala Pro Ala Thr Asp Gly Ser Gly Thr Ala Leu Gly Trp
    50                  55                  60

acc gtt gct tgg aaa aac aac tac cgt aac gct cac tct gct acc acc 240
Thr Val Ala Trp Lys Asn Asn Tyr Arg Asn Ala His Ser Ala Thr Thr
65                  70                  75                  80

tgg tct ggc cag tac gtt ggt ggt gct gaa gct cgt atc aac acc cag 288
Trp Ser Gly Gln Tyr Val Gly Gly Ala Glu Ala Arg Ile Asn Thr Gln
                85                  90                  95

tgg ctg ctg acc tct ggt acc acc gaa gct aac gct tgg aaa tct acc 336
Trp Leu Leu Thr Ser Gly Thr Thr Glu Ala Asn Ala Trp Lys Ser Thr
            100                 105                 110

ctg gtt ggt cac gac acg ttc acc aaa gtt aaa ccg tct gct gct tct 384
Leu Val Gly His Asp Thr Phe Thr Lys Val Lys Pro Ser Ala Ala Ser
        115                 120                 125

atc tag 390
Ile

130

<210> 2
<211> 651
<212> DNA
<213> Artificial Sequence

<220>
<221> CDS
<222> (97)..(534)

<400> 2
tctcgatccc gcgaaattaa tacgactcac tatagggaga ccacaacggt ttccctctag 60

aaataatttt gtttaacttt aagaaggaga tatacc atg tct ggt tct cat cat 114
Met Ser Gly Ser His His
1               5

cat cat cat cat gag gag cgc ggc gcg aat atg gct gaa gct ggt atc 162
His His His His Glu Glu Arg Gly Ala Asn Met Ala Glu Ala Gly Ile
            10                  15                  20

acc ggt act tgg tac aac cag ctg ggg tct acc ttc atc gtt acc gct 210
Thr Gly Thr Trp Tyr Asn Gln Leu Gly Ser Thr Phe Ile Val Thr Ala
        25                  30                  35

ggt gct gac ggt gca ctg acc ggt act tac gaa agc gct gtt ggt aac 258
Gly Ala Asp Gly Ala Leu Thr Gly Thr Tyr Glu Ser Ala Val Gly Asn
    40                  45                  50

gct gaa agc cgt tat gtt ctg acc ggt cgt tac gac tct gct ccg gct 306
Ala Glu Ser Arg Tyr Val Leu Thr Gly Arg Tyr Asp Ser Ala Pro Ala
55                  60                  65                  70

acc gac ggt tct ggt act gct ctg ggt tgg acc gtt gct tgg aaa aac 354
Thr Asp Gly Ser Gly Thr Ala Leu Gly Trp Thr Val Ala Trp Lys Asn
                75                  80                  85

aac tac cgt aac gct cac tct gct acc acc tgg tct ggc cag tac gtt 402
Asn Tyr Arg Asn Ala His Ser Ala Thr Thr Trp Ser Gly Gln Tyr Val
            90                  95                  100

ggt ggt gct gaa gct cgt atc aac acc cag tgg ctg ctg acc tct ggt 450
Gly Gly Ala Glu Ala Arg Ile Asn Thr Gln Trp Leu Leu Thr Ser Gly
        105                 110                 115

acc acc gaa gct aac gct tgg aaa tct acc ctg gtt ggt cac gac acg 498
Thr Thr Glu Ala Asn Ala Trp Lys Ser Thr Leu Val Gly His Asp Thr
    120                 125                 130

ttc acc aaa gtt aaa ccg tct gct gct tct atc tag ctgctgccac 544
Phe Thr Lys Val Lys Pro Ser Ala Ala Ser Ile
135                 140                 145

cgctgagcaa taactagcat aaccccttgg ggcctctaaa cgggtcttga ggggtttttt 604

gctgaaagga ggccgggaag gatgggtggt gtcttcagaa tcttgta 651

<210> 3

<211> 654

<212> DNA

<213> Artificial Sequence

<220>

<221> CDS

<222> (97)..(537)

<400> 3

tctcgatccc gcgaaattaa tacgactcac tatagggaga ccacaacggt ttccctctag 60

aaataatttt gtttaacttt aagaaggaga tatacc atg ggt tac cca tac gac 114

Met Gly Tyr Pro Tyr Asp

1               5

gtc cca gac tac gct gag gag cgc ggc gcg aat atg gct gaa gct ggt 162

Val Pro Asp Tyr Ala Glu Glu Arg Gly Ala Asn Met Ala Glu Ala Gly

            10                  15                  20

atc acc ggt act tgg tac aac cag ctg ggg tct acc ttc atc gtt acc 210

Ile Thr Gly Thr Trp Tyr Asn Gln Leu Gly Ser Thr Phe Ile Val Thr

        25                  30                  35

gct ggt gct gac ggt gca ctg acc ggt act tac gaa agc gct gtt ggt 258

Ala Gly Ala Asp Gly Ala Leu Thr Gly Thr Tyr Glu Ser Ala Val Gly

    40                  45                  50

aac gct gaa agc cgt tat gtt ctg acc ggt cgt tac gac tct gct ccg 306

Asn Ala Glu Ser Arg Tyr Val Leu Thr Gly Arg Tyr Asp Ser Ala Pro
55                  60                  65                  70

gct acc gac ggt tct ggt act gct ctg ggt tgg acc gtt gct tgg aaa    354
Ala Thr Asp Gly Ser Gly Thr Ala Leu Gly Trp Thr Val Ala Trp Lys
                75                  80                  85

aac aac tac cgt aac gct cac tct gct acc acc tgg tct ggc cag tac    402
Asn Asn Tyr Arg Asn Ala His Ser Ala Thr Thr Trp Ser Gly Gln Tyr
            90                  95                  100

gtt ggt ggt gct gaa gct cgt atc aac acc cag tgg ctg ctg acc tct    450
Val Gly Gly Ala Glu Ala Arg Ile Asn Thr Gln Trp Leu Leu Thr Ser
        105                 110                 115

ggt acc acc gaa gct aac gct tgg aaa tct acc ctg gtt ggt cac gac    498
Gly Thr Thr Glu Ala Asn Ala Trp Lys Ser Thr Leu Val Gly His Asp
    120                 125                 130

acg ttc acc aaa gtt aaa ccg tct gct gct tct atc tag ctgctgccac    547
Thr Phe Thr Lys Val Lys Pro Ser Ala Ala Ser Ile
135                 140                 145

cgctgagcaa taactagcat aaccccttgg ggcctctaaa cgggtcttga ggggtttttt 607

gctgaaagga ggccgggaag gatgggtggt gtcttcagaa tcttgta                654

<210> 4
<211> 17

<212> DNA
<213> Artificial Sequence

<220>
<223> DNA comprising a sequence of T7 RNA promoter

<400> 4
taatacgact cactata                                                    17

<210> 5
<211> 60
<212> DNA
<213> Artificial Sequence

<220>
<223> DNA comprising a sequence of T7 RNA promoter

<400> 5
tctcgatccc gcgaaattaa tacgactcac tatagggaga ccacaacggt ttccctctag  60

<210> 6
<211> 60
<212> DNA
<213> Artificial Sequence

<220>
<223> upstream primer for 1st PCR

<400> 6

tctcgatccc gcgaaattaa tacgactcac tatagggaga ccacaacggt ttccctctag 60

<210> 7
<211> 50
<212> DNA
<213> Artificial Sequence

<220>
<223> downstream primer for 1st PCR

<400> 7
tcctttcagc aaaaaacccc tcaagacccg tttagaggcc ccaaggggtt 50

<210> 8
<211> 27
<212> DNA
<213> Artificial Sequence

<220>
<223> upstream primer (Cy5-His) for 2nd PCR

<400> 8
ataccatgtc tggttctcat catcatc 27

<210> 9
<211> 24
<212> DNA
<213> Artificial Sequence

<220>

<223> upstream primer (FITC-HA) for 2nd PCR

<400> 9

ataccatggg ttacccatac gacg 24

<210> 10

<211> 24

<212> DNA

<213> Artificial Sequence

<220>

<223> downstream primer for 2nd PCR

<400> 10

gttattgctc agcggtggca gcag 24